# EUROPEAN PATENT APPLICATION

(11) **EP 4 445 824 A1**
(43) Date of publication of application: **16.10.2024**
(21) Application number: 22907845.6
(22) Date of filing: 08.12.2022
(51) Int. Cl.: A47L 23/20, A47B 61/04, F26B 21/00, F26B 7/00, F26B 9/00, F26B 3/02, A61L 2/07

(54) **SHOE CARE APPARATUS**

(30) Priority: 17.12.2021 KR 20210182008; 18.10.2022 KR 20220134249
(71) Applicant: LG Electronics Inc., Yeongdeungpo-gu Seoul 07336 (KR)
(72) Inventor: KIM, Jae Young, Seoul 08592 (KR); NAM, Bo Hyun, Seoul 08592 (KR); SONG, Seung Hyun, Seoul 08592 (KR)
(74) Representative: Mooser, Sebastian Thomas
(86) International application number: PCT/KR2022/019910
(87) International publication number: WO 2023/113378

(57) **Abstract**

Provided is a shoes care device configured to treat shoes for air circulation. The shoes care device comprise: an inner cabinet having an accommodation space configured to accommodate shoes, wherein a shelf holder is provided on an inner side wall; an outlet provided in a bottom surface of the inner cabinet to allow air inside the inner cabinet is suctioned; a connection path constituting a flow path through which the air in the accommodation space is discharged from the inner cabinet through the suction port and then introduced back into the accommodation space; a blowing part disposed in the connection path to blow air; a dehumidifying part disposed in the connection path to dehumidify air; and an auxiliary shelf installed to be mounted on the shelf holder so that the shoes can be seated on an upper surface of the auxiliary shelf.

## Description

### TECHNICAL FIELD

The present invention relates to a shoes care device, and more particular, to a shoes care device which treats shoes by air circulation.

### BACKGROUND

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device 1, which removes germs and odors by performing a predetermined treatment on the shoes so that a user can comfortably wear the shoes all the times.

For the shoes care device, Korean Patent No. 1037245 (hereinafter, referred to as "Prior Document 1") discloses "Apparatus for sterilization disposal of shoes", which includes a main body, an ultraviolet emission module, and a deodorization module.

According to Prior Document 1 above, the shoes are disposed in a sterilization chamber of the main body, and the ultraviolet emission module is driven to remove germs and odors of the shoes. Furthermore, the air in the sterilization chamber is inhaled into a ventilation pipe and discharged to the outside of the main body through an exhaust port through the deodorization module.

Here, the deodorization module includes a deodorization column made up of materials such as zeolite, activated carbon, and charcoal, and removes contaminants from the air discharged from the inside of the main body to the outside by the deodorization column.

According to Prior Document 1 above, the air from which moisture has been removed by a deodorization module including zeolite and activated carbon can be discharged to the outside of the apparatus for sterilization disposal of shoes.

However, in Prior Document 1 above, since the air is discharged to the outside of the apparatus for sterilization disposal of shoes, the air that has not sufficiently removed moisture or odors may be discharged to the outside of the apparatus for sterilization disposal of shoes, and such air may be discharged to the inside where the wearer resides.

In addition, Korean Patent Unexamined Publication No. 10-2000-0009653 (hereinafter referred to as "Prior Document 2") discloses "The shoes cabinet for sanitation", which includes a main body, a far infrared radiation part, a circulation fan, an air circulation passage, and a sanitary filter portion.

According to Prior Document 2 above, while storing shoes in shoe closet, hygiene treatments such as dehumidification, sterilization, and deodorization can be performed on the shoes by far-infrared rays and a filter during the storing of the shoes.

Here, since the sanitary filter portion is filled with excellent adsorptive materials such as charcoal, it can serve to adsorb moisture in the process of ventilating air and filter bacteria, as well as to capture malodorous substances.

According to Prior Document 2 above, the air is circulated by a circulating fan in a shoe closet, and the sanitary filter portion is disposed on a circulation path of the air to remove germs and odors in the air

However, since Prior Document 2 above does not consider a technology to prevent the reduction of the performance of the sanitary filter portion configured to remove moisture or odors, a user may not be satisfied because the shoes are not properly treated at the time of using a shoes care device.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device cannot properly solve such a task.

In addition, when designing and manufacturing devices including a dehumidifier such as zeolite, it is necessary to consider how the dehumidification efficiency of a dehumidifying material can be further improved, whether the dehumidifying material can be effectively regenerated, whether water vapor generated during the use of the shoes care device and the regeneration of the dehumidifying material can be effectively removed or managed, whether moisture is left in an unintended area during use of the shoes care device and the regeneration of the dehumidifying, whether components are properly disposed in a limited space, and where the devices provides excellent use convenience. There is a need to develop a shoes care device that takes these matters into consideration.

In addition, the reduction of manufacturing costs, the convenience of manufacturing and assembling each component, and the convenience of maintenance needs to be considered in the development of the shoes care device.

### DISCLOSURE

### TECHNICAL PROBLEM

An object to be achieved by the present invention is to solve the aforementioned problems caused by a shoes care device which treats shoes by air circulation.

Specifically, an object to be achieved by the present invention is to provide a shoes care device which can ensure proper performance for shoes treatment at all times by performing dehumidification and deodorization on the shoes using a dehumidifying material to refresh the shoes and regenerating the used dehumidifying material.

An object to be achieved by the present invention is to provide a shoes care device which prevents air used for dehumidification and deodorization of shoes from being exposed to a user by an air circulation structure capable of dehumidifying the air inside an inner cabinet where the shoes are placed by using a dehumidifying material and supplying the dehumidified air again to the inner cabinet.

An object to be achieved by the present invention is to provide a shoe care device that allows a plurality of shoes to be treated within only one inner cabinet, allowing for more diverse usage.

The technical objects of the present disclosure are not restricted to those set forth Here, and other unmentioned technical objects will be clearly understood by one of ordinary skill in the art to which the present disclosure pertains by referencing the detailed description of the present disclosure given below.

### TECHNICAL SOLUTION

In order to achieve the above and other objects, a shoes care device according to one aspect of the present invention is configured not only to perform dehumidification and deodorization of shoes using a dehumidifying part but also to regenerate the used dehumidifying part. Specifically, the shoes care device is configured not only to collect moisture and bacteria in air ventilated by the dehumidifying part in a module chamber but also to heat and regenerate the dehumidifying part in the module chamber.

In addition, according to one aspect of the present invention, the shoes care device is configured such that the air used for dehumidifying and deodorizing the shoes has an air circulation structure inside the shoes care device. Specifically, a connection path through which air is circulated is formed between a outlet and a nozzle disposed inside an inner cabinet, respectively.

In addition, a shoe care device according to an aspect of the present disclosure is configured to treat a plurality of shoes within only one inner cabinet. Specifically, the shoe care device is configured to be used by mounting an auxiliary shelf on a shelf holder provided in the inner cabinet.

In addition, the shoe care device according to an aspect of the present disclosure may be configured such that an auxiliary shelf that is mounted to be detachable from the shelf holder.

In addition, the shoe care device according to an aspect of the present disclosure may be configured such that an auxiliary isolation rib is provided on the upper surface of the auxiliary shelf.

In addition, the shoe care device according to an aspect of the present disclosure may be configured such that an auxiliary isolation ribs is provided in a diagonal direction.

In addition, the shoe care device according to an aspect of the present disclosure may configured such that an auxiliary order rib is provided along the peripheral surface of the auxiliary shelf.

In addition, the shoe care device according to an aspect of the present disclosure may be configured such that the central portion of the auxiliary shelf is convex.

In addition, the shoe care device according to an aspect of the present disclosure may be configured such that, when the auxiliary shelf is mounted in the inner cabinet, the nozzle duct is inserted into a cutout portion of the auxiliary shelf.

In addition, the shoe care device according to an aspect of the present disclosure may be configured such that, in the state in which the auxiliary shelf is mounted in the inner cabinet, the upper discharge port discharges air to the upper portion of the auxiliary shelf from, and the lower discharge port discharges air to the lower portion of the auxiliary shelf.

In addition, the shoe care device according to an aspect of the present disclosure may be configured such that the upper surface of the nozzle body portion and the lower surface of the auxiliary shelf are magnetically attached to each other.

In addition, the shoe care device according to an aspect of the present disclosure may be configured such that the lower surface portion in front of the cutout portion that is in close contact with the upper surface of the nozzle duct, is inclined.

In addition, the shoe care device according to an aspect of the present disclosure is configured such that a handle groove is provided in the auxiliary shelf portion corresponding to the upper portion of the nozzle handle.

In addition, the shoe care device according to an aspect of the present disclosure may be configured such that an accommodation groove is provided on the inner surface of the door to accommodate the auxiliary shelf.

In addition, the shoe care device according to an aspect of the present disclosure may be configured such that the lower portion of the accommodation groove is spaced apart from the auxiliary shelf to a certain extent.

In addition, the shoe care device according to an aspect of the present disclosure may be configured such that the auxiliary shelf and the accommodation groove are magnetically attached to each other.

In addition, the shoe care device according to an aspect of the present disclosure may be configured such that the auxiliary shelf is accommodated in the accommodation groove such that the cutout position is located on the lower side.

In addition, the shoe care device according to an aspect of the present disclosure may be configured such that the insertion protrusion of the auxiliary shelf is inserted into the insertion groove of the door in the state in which the auxiliary shelf is accommodated in the accommodation groove.

In addition, the shoe care device according to an aspect of the present disclosure may be configured such that the insertion rib provided inside the insertion groove supports the insertion protrusion of the auxiliary shelf.

In addition, the shoe care device according to an aspect of the present disclosure may be configured such that a certain amount of free space may be provided in the insertion groove portion where the insertion protrusion is inserted.

In addition, the shoe care device according to an aspect of the present disclosure may be configured such that the insertion protrusion inserted into the insertion groove may be inserted sufficiently deeply compared to its width.

In addition, the shoe care device according to an aspect of the present disclosure may be configured such that the auxiliary shelf includes metal that can be magnetically attached to the accommodation groove.

The technical objects of the present disclosure are not restricted to those set forth Here, and other unmentioned technical objects will be clearly understood by one of ordinary skill in the art to which the present disclosure pertains by referencing the detailed description of the present disclosure given below.

### BRIEF DESCRIPTION OF THE DRAWINGS

FIG. 1a is a perspective view illustrating a shoes care device according to one embodiment of the present invention.
FIG. 1b is a perspective view of the shoes care device of FIG. 1a, when viewed from another direction, illustrating a state in which a door is opened.
FIG. 2a is a perspective view illustrating a state in which a part of the door and an outer cabinet are removed from the shoes care device of FIG. 1b.
FIG. 2b is a perspective view illustrating a state in which the shoes care device illustrated in FIG. 2a is viewed from another direction.
FIG. 3 is a front view illustrating a state in which a door is removed from the shoes care device illustrated in FIG. 1b. FIG. 3 illustrates shoes accommodated in an inner cabinet together
FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.
FIG. 5 is a perspective view illustrating a state in which the door, the outer cabinet, and the inner cabinet are removed from the shoes care device according to one embodiment of the present invention.
FIG. 6 is a perspective view illustrating a part of a machine room of the shoes care device illustrated in FIG. 5.
FIG. 7a is a view illustrating a steam valve according to one embodiment of the present invention, and illustrating a connection relationship considering the movement of steam.
FIG. 7b is an exploded perspective view illustrating the steam valve illustrated in FIG. 7a.
FIG. 8a is a cross-sectional view taken along line D-D' of the shoes care device illustrated in FIG. 3. FIG. 8a illustrates a state in which the door is included in the shoes care device, and does not illustrate the shoes.
FIG. 8b is a view illustrating a state in which a main shelf is removed from the shoes care device illustrated in FIG. 8a.
FIG. 9 is a cross-sectional view taken along line E-E' of the shoes care device illustrated in FIG. 3. FIG. 9 illustrates a state in which the door is included in the shoes care device.
FIG. 10a is an exploded perspective view illustrating a drying module in the shoes care device according to one embodiment of the present invention. FIG. 10b is an exploded perspective view illustrating a partial configuration of the drying module at a part to which a damper is coupled.
FIG. 11 is a diagram illustrating a connection relationship between components and a flow of fluid in the shoes care device according to one embodiment of the present invention.
FIG. 12 is a view illustrating a state in which an auxiliary shelf is installed in the shoes care device according to one embodiment of the present invention.
FIG. 13a is a cross-sectional view illustrating an inner surface of the door illustrated in the shoes care device according to one embodiment of the present invention.
FIGS. 13b to 13e are a view illustrating a insertion groove and a insertion protrusion in more detail in the shoes care device according to one embodiment of the present invention.
FIG. 14 is a perspective view illustrating the auxiliary shelf in the inner surface of the door according to one embodiment of the present invention.
FIG. 15 is a view illustrating the auxiliary shelf and a nozzle duct in the shoes care device according to one embodiment of the present invention.
FIG. 16 is a view illustrating a state in which the nozzle duct is coupled to a cross section taken along line Cc-C'c of the auxiliary shelf illustrated in FIG. 14.
FIG. 17 is a view exemplarily illustrating a state in which the shoes are settled on the auxiliary shelf in the shoes care device according to one embodiment of the present invention.

### DETAILED DESCRIPTION

Hereinafter, exemplary embodiments disclosed herein will be described in detail with reference to the accompanying drawings, and like reference numerals designate like elements, and redundant description thereof will be omitted. Suffixes "module" and "unit or portion" for elements used in the following description are merely provided for facilitation of preparing this specification, and thus they are not granted a specific meaning or function. Wherever possible, the same reference numbers will be used throughout the drawings to refer to the same or like parts. In the following description, known functions or structures, which may confuse the substance of the present disclosure, are not explained. The accompanying drawings are used to help easily explain various technical features and it should be understood that the exemplary embodiments presented herein are not limited by the accompanying drawings. As such, the present disclosure should be construed to extend to any alterations, equivalents and substitutes in addition to those which are particularly set out in the accompanying drawings.

Although the terms first, second, and the like, may be used herein to describe various elements, these elements should not be limited by these terms. These terms are generally only used to distinguish one element from another

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present.

The singular expressions include plural expressions unless the context clearly dictates otherwise.

It should be understood that the terms "comprises," "comprising," "includes," "including," "containing," "has," "having" or any other variation thereof specify the presence of stated features, integers, steps, operations, elements, and/or components, but do not preclude the presence or addition of one or more other features, integers, steps, operations, elements, and/or components.

A first direction (X direction), a second direction (Y direction), and a third direction (Z direction) described in one embodiment of the present invention may be directions orthogonal to each other.

Each of the first direction (X direction) and the second direction (Y direction) may be a direction parallel to the horizontal direction, and the third direction (Z direction) may be a direction parallel to the vertical direction. When the first direction (X direction) is parallel to a left-right direction, the second direction (Y direction) may be parallel to a front-rear direction. When the first direction (X direction) is parallel to the front-rear direction, the second direction (Y direction) may be parallel to the left-right direction.

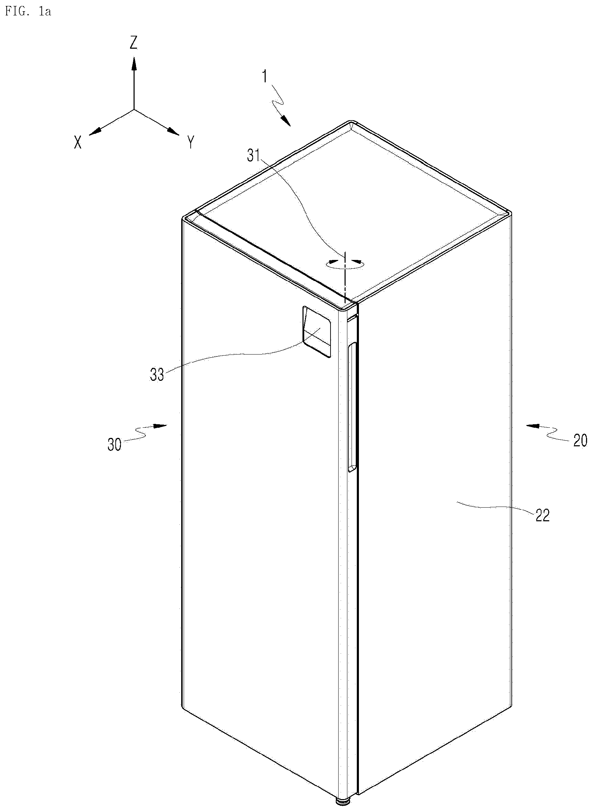

FIG. 1a is a perspective view illustrating a shoes care device according to one embodiment of the present invention.

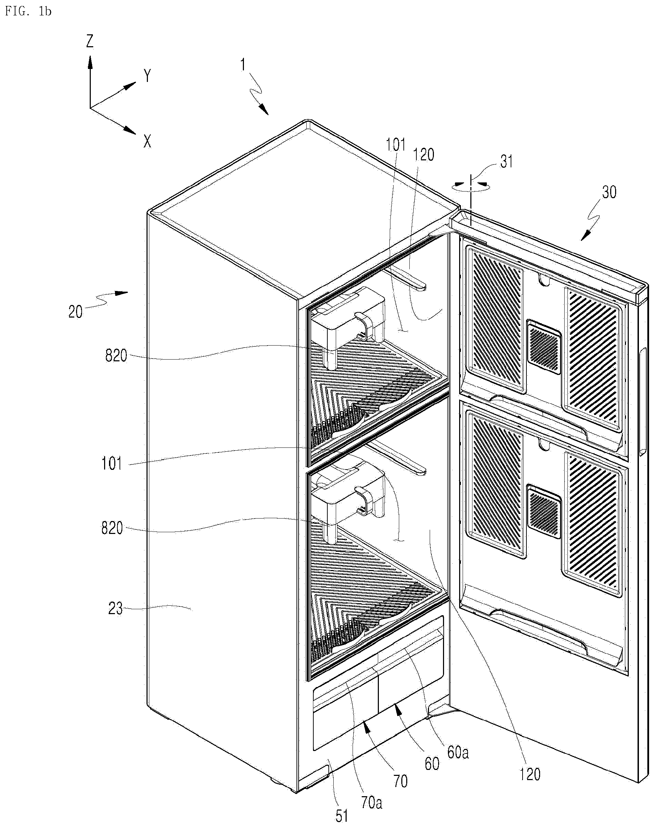

FIG. 1b is a perspective view of the shoes care device of FIG. 1a, when viewed from another direction, illustrating a state in which a door is opened.

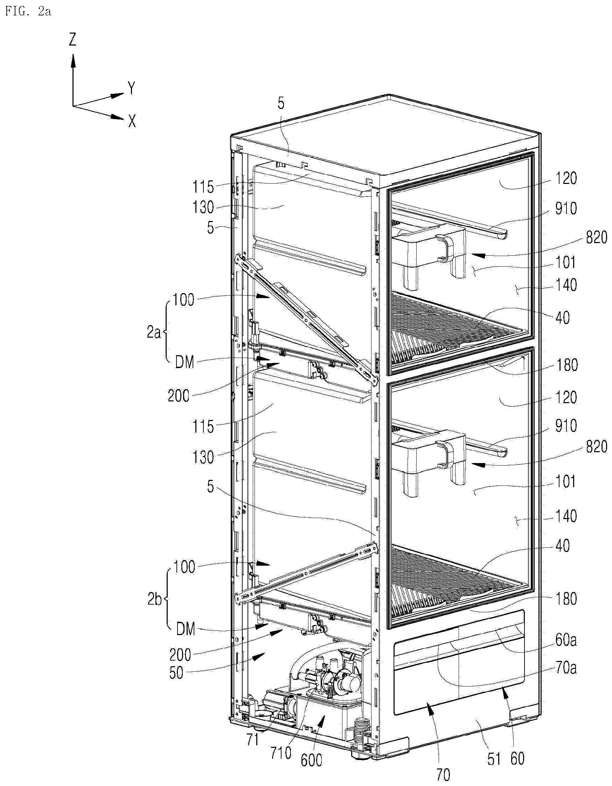

FIG. 2a is a perspective view illustrating a state in which a part of the door and an outer cabinet are removed from the shoes care device of FIG. 1b.

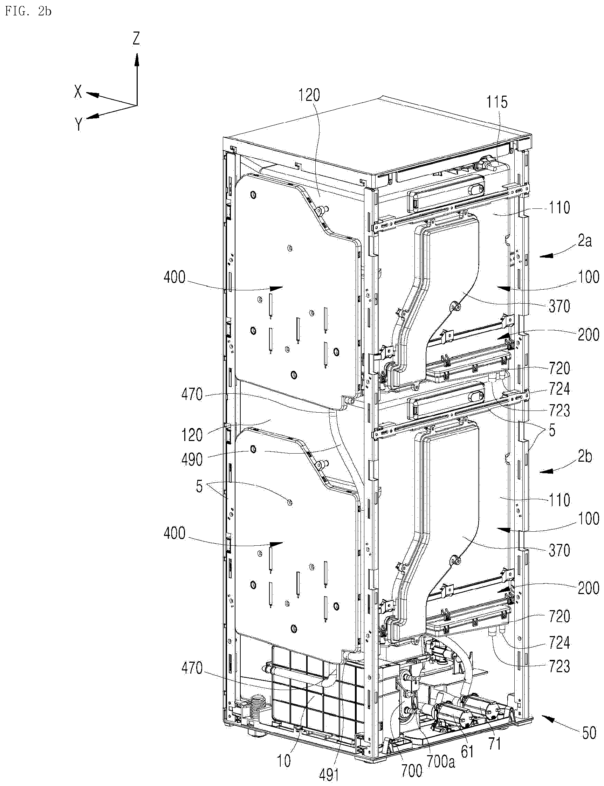

FIG. 2b is a perspective view illustrating a state in which the shoes care device illustrated in FIG. 2a is viewed from another direction.

A shoes care device 1 according to one embodiment of the present invention may include an outer cabinet 20, a door 30, an inner cabinet 100, and a machine room 50. The shoes care device 1 may include a main frame 5.

The outer cabinet 20 and the door 30 may form an overall appearance of the shoes care device 1. The outside of the shoes care device 1 may be formed in a hexahedral shape. That is, while the outer cabinet 20 and the door 30 are coupled to each other and the door 30 is closed, the appearance of the shoes care device 1 may be formed in a hexahedral shape. However, the shoes care device 1 according to one embodiment of the present invention is not limited to such a shape and may have various three-dimensional shapes.

When the door 30 forms the front of the shoes care device 1, the outer cabinet 20 may form an upper side surface, a left-side surface, a right-side surface, a rear surface, and bottom surfaces of the shoes care device 1.

The main frame 5 may form an overall framework of the shoes care device 1. The main frame 5 may have a hexahedral structure.

The outer cabinet 20 may be detachably fixed to the main frame 5.

The outer cabinet 20 may include an outer rear plate 21, a first outer side plate 22, and a second outer side plate 23.

The outer back plate 21, the first outer side plate 22, and the second outer side plate 23 may be formed integrally with each other, or the outer back plate 21, the first outer side plate 22, and the second outer side plate 23 may be individually formed.

The outer rear plate 21 forms a vertically erected wall surface. The outer rear plate 21 may form a surface orthogonal to the first direction (X direction). The outer rear plate 21 may form a rear wall surface in the first direction (X direction) on the outer cabinet 20. The outer rear plate 21 may form a rear surface in the first direction (X direction) in the shoes care device 1. The outer rear plate 21 may form an entire outer rear surface of the shoes care device 1.

The first outer side plate 22 and the second outer side plate 23 form vertically erected wall surfaces, respectively, and form opposing wall surfaces facing each other.

The first outer side plate 22 is disposed at any one side with respect to a reference plane RP that is parallel to the first direction (X direction) as a horizontal direction and is a vertical plane. The second outer side plate 23 is disposed on the opposite side of the first outer side plate 22 with respect to the reference plane RP. The first outer side plate 22 may form a left wall surface of the outer cabinet 20, and the second outer side plate 23 may form a right wall surface of the outer cabinet 20.

The outer cabinet 20 may be disposed outside the inner cabinet 100 and the machine room 50 to form an outer wall surface of the machine room 50. When a separate cabinet for the machine room 50 is not provided in the shoes care device 1, the outer cabinet 20 may form a wall separating the machine room 50 from the outside.

The door 30 is configured to open and close the inside of the shoes care device 1. The door 30 may form any one surface of the shoes care device 1. The door 30 may form a left side or a right side of the shoes care device 1, or may form a front side of the shoes care device 1.

In the shoes care device 1, the door 30 may be hinge-coupled.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoes care device 1, the door 30 may be configured to be rotatable bidirectionally around a rotation axis 31 in the vertical direction.

In one embodiment, the shoes care device 1 may include one door 30. In another embodiment, the shoes care device 1 may include two or more doors.

When two doors are provided in the shoes care device 1, each door may be individually rotated around each rotation axis.

The first direction (X direction) described in one embodiment of the present invention may be parallel to or substantially parallel to the horizontal direction.

In one embodiment, the first direction (X direction) may be a direction from the rear of the shoes care device 1 to the front.

Hereinafter, except for a particularly limited case, it will be described that the door 30 is formed in a front side of the shoes care device 1. That is, a surface on which the door 30 is formed in the shoes care device 1 is described as a front surface of the shoes care device 1.

Furthermore, hereinafter, except for a particularly limited case, it will be described that the first direction (X direction) is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

A plurality of shoes S may be disposed together in the accommodation space 101 of one inner cabinet 100.

The inner cabinet 100 may be fixed to the main frame 5.

The inner cabinet 100 has a predetermined size along the first direction (X direction), the second direction (Y direction), and the third direction (Z direction).

The inner cabinet 100 is formed in the shape of a box opened to any one side. The inner cabinet 100 may be formed in a form opened to the front side of the shoes care device 1. The inner cabinet 100 may be configured to include a main opening 140. The main opening 140 may be provided to open the front side of the inner cabinet 100 in the first direction (X direction). The shoes may be disposed inside the inner cabinet 100 or withdrawn from the inner cabinet 100 through the main opening 140.

The main opening 140 of the inner cabinet 100 may be closed or opened by the door 30.

The inner cabinet 100 may include an inner rear plate 110, a first inner side plate 120, a second inner side plate 130, and an inner upper plate 115.

The inner rear plate 110, the first inner side plate 120, the second inner side plate 130, and the inner upper plate 115 may be formed integrally with each other. The inner cabinet 100 may be made of a single material and be formed by injection molding.

The inner rear plate 110 forms a vertically erected wall surface. The inner rear plate 110 may form a surface orthogonal to the first direction (X direction). The inner rear plate 110 may form a rear wall surface of the inner cabinet 100 in the first direction (X direction). The inner rear plate 110 may be formed parallel to the outer rear plate 21.

The first inner side plate 120 and the second inner side plate 130 form vertically erected wall surfaces, respectively, and form opposing wall surfaces facing each other.

The first inner side plate 120 is dispose on either side with respect to the reference plane RP that is parallel to the first direction (X direction) as the horizontal direction and is a vertical surface. The second inner side plate 130 is disposed on the opposite side of the first inner side plate 120 with respect to the reference plane RP. The first inner side plate 120 forms a left wall surface of the inner cabinet 100, and the second inner side plate 130 forms a right wall surface of the inner cabinet 100.

The first inner side plate 120 may be formed parallel to the first outer side plate 22, and the second inner side plate 130 may be formed parallel to the second outer side plate 23.

In one embodiment of the present invention, the inner cabinet 100 may have the form where a lower part thereof is opened. Accordingly, the inner cabinet 100 includes a lower opening 150 formed by opening the lower part thereof. When the inner cabinet 100 is formed in a hexahedral shape on the whole, the lower opening 150 may be formed large to form all or most of a lower surface of the inner cabinet 100 (see FIGS. 53 and 54).

However, the shoes care device 1 according to one embodiment of the present invention is not used in a state in which an entire lower part of the inner cabinet 100 is opened, but is used in a state in which the inner cabinet 100 and a module housing 200 are coupled to each other so that the lower opening 150 of the inner cabinet 100 is shielded by the module housing 200. That is, the shoes care device 1 is used so that an upper surface of the module housing 200 forms a bottom surface of the accommodation space 101 of the inner cabinet 100. A further explanation thereof will be described below.

A main shelf 40 may be provided in the inside 101 of the inner cabinet 100. The main shelf 40 may be formed such that the shoes S are settled on an upper surface thereof.

The main shelf 40 may be formed in the form of a plate with a predetermined area, or may be formed in the form of a grill where multiple bars are spaced apart from each other.

One main shelf 40 may be provided, or a plurality of main shelves 40 may be provided.

The main shelf 40 may have a substantially flat plate shape and be disposed on a bottom surface of the inner cabinet 100. The main shelf 40 is settled on an upper side of a module cover 202 of the inner cabinet 100. The main shelf 40 may be disposed on the upper side of the module cover 202 of the inner cabinet 100 in a stacked form.

The main shelf 40 is detachable from the inner cabinet 100, and when the main shelf 40 is withdrawn from the inner cabinet 100, the upper surface of the module housing 200 is exposed.

The main shelf 40 may have a rectangular shape when viewed in a plan view. The size of the main shelf 40 may be a size corresponding to the bottom of the accommodation space 101 of the inner cabinet 100. That is, when the main shelf 40 is disposed inside the inner cabinet 100, the main shelf 40 may form all or most of the bottom of the accommodation space 101 of the inner cabinet 100.

In one embodiment, the machine room 50 may be provided in a lower side of the inner cabinet 100. In the machine room 50, some of the components that constitutes the shoes care device 1 may be accommodated, and, in this case, the components that are accommodated in the machine room 50 may be fixed to a main frame 5 or to the inner cabinet 100 or the outer cabinet 20.

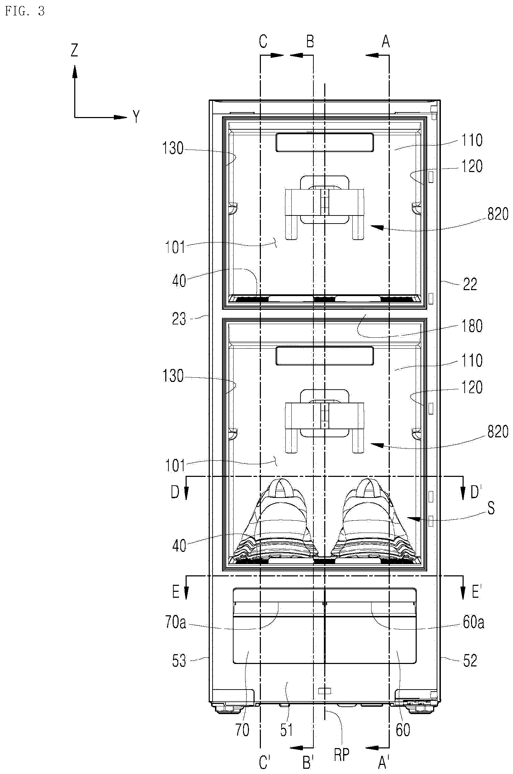

FIG. 3 is a front view illustrating a state in which a door is removed from the shoes care device illustrated in FIG. 1b. FIG. 3 illustrates shoes accommodated in an inner cabinet together

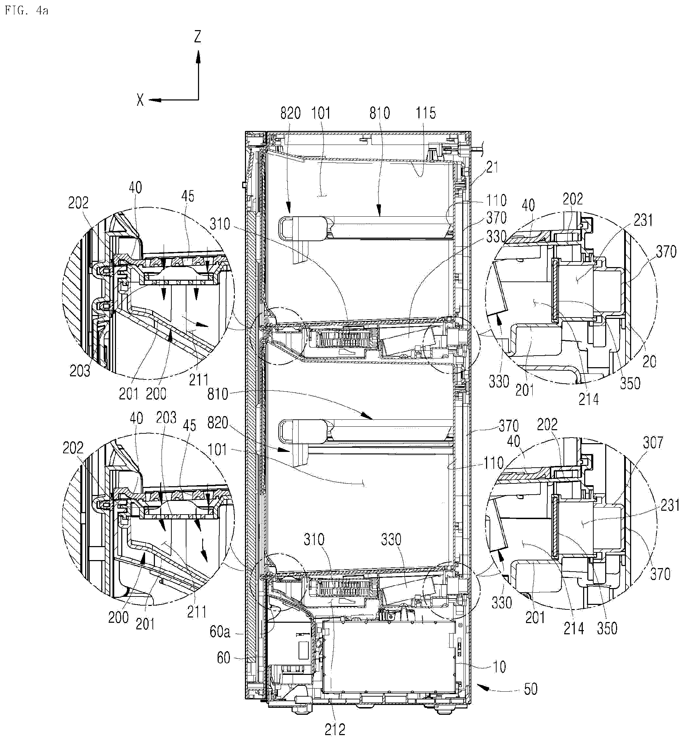

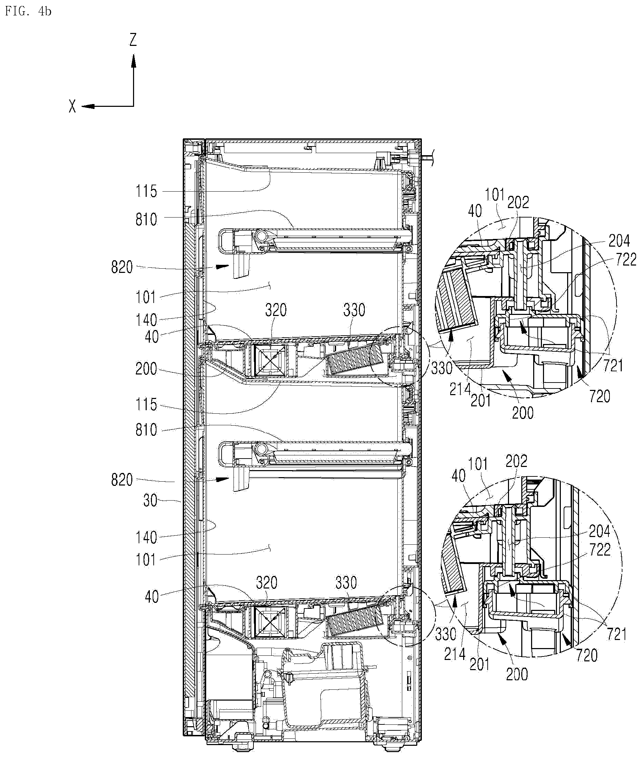

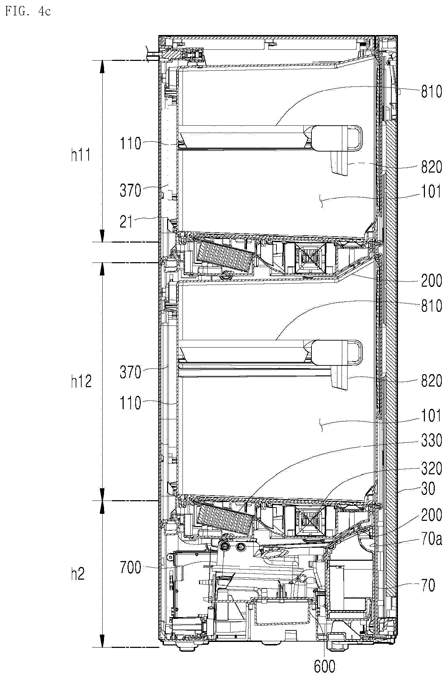

FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.

The shoes care device 1 according to one embodiment of the present invention includes management devices 2a and 2b. In the shoes care device 1, a plurality of management devices 2a and 2b may be provided. In one embodiment, the shoes care device 1 may include a first management device 2a and a second management device 2b. That is, the shoes care device 1 may include two separate management devices 2a and 2b.

The 'management device' described in the present invention may refer to a 'first management device 2a' and a'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoes care device 1, the door 30 closes the main opening 140 of the first management device 2a and also closes the main opening 140 of the second management device 2b. That is, the door 30 may simultaneously seal the accommodation space 101 of the inner cabinet 100 of the first management device 2a and the accommodation space 101 of the inner cabinet 100 of the second management device 2b. In this case, the accommodation space 101 of the inner cabinet 100 of the first management device 2a and the accommodation space 101 of the inner cabinet 100 of the second management device 2b may be configured such that they do not communicate with each other.

As described above, in one embodiment of the present invention, the accommodation space 101 of the inner cabinet 100 of the first management device 2a and the accommodation space 101 of the inner cabinet 100 of the second management device 2b may form independent spaces, and may form blocked spaces (not communicating with each other). Accordingly, the temperature and humidity of the accommodation space 101 of the first management device 2a and the temperature and humidity of the accommodation space 101 of the second management device 2b may be controlled differently from each other.

The management devices 2a and 2b may be configured to include a connection path F10. The management devices 2a and 2b may be configured to include a blowing part 310 and a dehumidifying part 330. The management devices 2a and 2b may include a outlet 203 and a nozzle 820.

The management devices 2a and 2b may include the module housing 200 forming a connection path F10.

The management devices 2a and 2b may include a regeneration path F20. The management devices 2a and 2b may include a heating part 320.

The management devices 2a and 2b may include a conversion flow path FlOa.

The management devices 2a and 2b may include a damper 350.

The shoes care device 1 may include a steam generator 700 and a steam valve 710.

The shoes care device 1 may include a sump 600, a water supply tank 60, and a drain tank 70.

Since all or part of the outer cabinet 20 may be spaced apart from the inner cabinet 100, a predetermined gap may be formed between the inner cabinet 100 and the outer cabinet 20.

In a space between the inner cabinet 100 and the outer cabinet 20, components constituting the shoes care device 1 may be provided, and various flow paths constituting the shoes care device 1 may be provided. In one embodiment of the present invention, part of the connection path F10 may be provided between the inner cabinet 100 and the outer cabinet 20, and part of the regeneration path F20 may be provided between the inner cabinet 100 and the outer cabinet 20.

A dry air duct 370 forming the connection path F10 may be provided between the outer rear plate 21 and the inner rear plate 110. Furthermore, a condenser 400 forming the regeneration path F20 may be provided between the outer rear plate 21 and the inner rear plate 110.

The connection path F10 forms a flow path of a fluid

The connection path F10 forms a passage through which air and/or condensed water inside the shoes care device 1 moves

The dehumidifying part 330 is disposed inside the connection path F10 and includes a dehumidifying material. The dehumidifying part 330 may be entirely formed of the dehumidifying material, or a part thereof may be formed of the dehumidifying material. A further explanation of the dehumidifying part 330 will be described below.

According to one embodiment of the present invention, the shoes care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the shoes care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the accommodation space 101 of the inner cabinet 100, and forms an inlet of the module housing 200 through which the air of the accommodation space 101 of the inner cabinet 100 is suctioned into the module housing 200. The outlet 203 may be disposed below the main shelf 40. In this case, the main shelf 40 may be formed so as not to block the air in the accommodation space 101 from being sucked into the outlet 203. To this end, when the main shelf 40 is formed in a plate shape, a plurality of holes 45 penetrating in the vertical direction may be formed in the main shelf 40 so as to move the air.

In the shoes care device 1, the inner cabinet 100 and the machine room 50 may form a space separated from each other. Furthermore, the module housing 200 that is part of the management devices 2a and 2b may be provided between the inner cabinet 100 and the machine room 50.

The inner cabinet 100, the module housing 200, and the machine room 50 are provided inside the shoes care device 1 according to one embodiment of the present invention.

The inner cabinet 100, the module housing 200, and the machine room 50 may be continuously arranged from the upper side to the lower side. When the shoes care device 1 according to one embodiment of the present disclosure includes the first management device 2a and the second management device 2b, the first management device 2a may be disposed above the second management device 2b. That is, the first management device 2a, the second management device 2b, and the machine room 50 may be continuously arranged from the upper side to the lower side.

Since the first management device 2a and the second management device 2b include the inner cabinet 100 and the module housing 200, respectively, they may be continuously arranged in an order of the inner cabinet 100 of the first management device 2a, the module housing 200 of the first management device 2a, the inner cabinet 100 of the second management device 2b, the module housing 200 of the second management device 2b, the machine room 50 from the upper side to the lower side.

The inner cabinet 100 may form a space that mainly accommodates an article (shoes S) to be managed, and the module housing 200 and the machine room 50 may form a space that mainly accommodates components for an operation of the shoes care device 1.

In the shoes care device 1 according to one embodiment of the present invention, the blowing part 310, the dehumidifying part 330 (and the dehumidifying material 331), and the heating part 320 may be accommodated inside the module housing 200.

In addition, the machine room 50 may be configured to accommodate a controller 10, the sump 600, the steam generator 700, and the steam valve 710 therein. Furthermore, the machine room 50 may be configured to accommodate the water supply tank 60 and the drain tank 70.

Among the components constituting the management devices 2a and 2b, components not included in the module housing 200 may be fixedly coupled to the inner cabinet 100 and the outside of the module housing 200 or may be fixedly coupled to the main frame 5.

Components coupled to or accommodated in the machine room 50 may be fixedly coupled to the machine room 50.

The machine room 50 may include a first wall 51.

A first wall 51 forms one wall surface of the machine room 50. The first wall 51 may be erected in the vertical direction, or may be erected in the substantially vertical direction. In one embodiment, the first wall 51 may form a wall surface orthogonal to or inclined with the first direction (X direction).

The first wall 51 may form a front wall surface of the machine room 50, a left wall of the machine room 50, or a right wall of the machine room 50.

The machine room 50 may include a second wall 52 and a third wall 53. The second wall 52 and the third wall 53 form opposite wall surfaces facing each other in the machine room 50. The second wall 52 and the third wall 53 may be erected in the vertical direction, or may be erected in the substantially vertical direction.

When the first wall 51 forms a front wall of the machine room 50, the second wall 52 may form a left wall of the machine room 50, and the third wall 53 may form a right wall of the machine room 50.

The first wall 51 may be formed integrally with the inner cabinet 100, and the second wall 52 and the third wall 53 may be formed integrally with the outer cabinet 20, respectively.

Each of the water supply tank 60 and the drain tank 70 may be formed in the form of a container for accommodating water.

The water supply tank 60 may be configured to store water supplied into the shoes care device 1 inside. The water supply tank 60 may be configured to store water supplied into the steam generator 700 inside.

In order to supply water from the water supply tank 60 into the shoes care device 1, a water pump 61 may be connected to the water supply tank 60. The water supply tank 60 for moving water and the first water pump 61 may be connected by a pipe, a hose, etc.

The drain tank 70 may be configured to store water discharged from the shoes care device 1 inside. The drain tank 70 may store water condensed inside the shoes care device 1. The drain tank 70 may be configured to store water drained from the sump 600.

In order to discharge water to the drain tank 70, a water pump 71 (a second water pump) may be connected to the drain tank 70. The drain pipe 70 for moving water and the second water pump 71 may be connected by a pipe, a hose, etc.

The water supply tank 60 and the drain tank 70 may be coupled to the machine room 50 to get exposed from the outside of one wall surface of the machine room 50.

The water supply tank 60 and the drain tank 70 may be disposed in front of the machine room 50.

The water supply tank 60 and the drain tank 70 may form one wall surface of the machine room 50 along with the first wall 51. When the first wall 51 forms a front surface of the machine room 50, the water supply tank 60 and the drain tank 70 may be exposed from a front side of the machine room 50, and may be coupled to the machine room 50 to get exposed from the outside of the first wall 51.

As the water supply tank 60 and the drain tank 70 are exposed to the outside of the first wall 51, a user may inject water into the water supply tank 60 or discharge water from the drain tank 70.

The water supply tank 60 and the drain tank 70 may be configured to be detachable from the machine room 50. The water supply tank 60 and the drain tank 70 may be detached from the first wall 51. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

Each of the water supply tank 60 and the drain tank 70 may be configured to be separated from the machine room 50 in an outer direction of the first wall 51.

The controller 10 may be configured to control operations of each component in connection with each component constituting the shoes care device 1.

In order to control the controller 10, the shoes care device 1 may be provided with a storage medium in which an application program is stored, and the controller 10 may be configured to control the shoes care device 1 by driving an application program according to information input to the shoes care device 1 and information output from the shoes care device 1.

The controller 10 may control the first management device 2a and the second management device 2b constituting the shoes care device 1 to operate individually. The controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the controller 10 may control the first management device 2a and the second management device 2b to interwork with each other.

The door 30 may be disposed on either the inner cabinet 100 or the machine room 50 on the same side as the first wall 51. When the door 30 forms the front surface of the shoes care device 1, the first wall 51 forms the front surface of the machine room 50, and the door 30 is disposed directly outside the first wall 51.

In one embodiment of the present invention, the door 30 may be configured to open and close the inner cabinet 100 and further expose or shield the front surface of the machine room 50.

The door 30 may be configured to expose or shield the inner cabinet 100, the water supply tank 60, and the drain tank 70.

As described above, in the shoes care device 1, the door 30, the water supply tank 60, and the drain tank 70 are formed on the same side, and when the door 30 is opened, the water supply tank 60 and the drain tank 70 may be exposed and separated from the shoes care device 1.

In the arrangement explained above, even if left and right sides and a rear side of the shoes care device 1 are blocked by other goods or structures, the door 30 may be opened on a front side of the shoes care device 1, and the water supply tank 60 and the drain tank 70 can be separated from or coupled again to the shoes care device 1.

A control panel 33 for controlling the shoes care device 1 is provided on an outer side of the door 30. The control panel 33 may be formed of a touch screen. A control unit (controller 10) is provided in the inner space of the door 30 to control each component of the shoes care device 1 in connection with the control panel 33. The controller 10 may be provided inside the machine room 50.

As illustrated in FIGS. 1a and 1b, in one embodiment, the door 30 may be configured to simultaneously expose or shield the inner cabinet 100 and the machine room 50.

In another embodiment, the door 30 may be configured to open and close only the inner cabinet 100. In this case, the machine room 50 may not be shielded by the door 30. Furthermore, in this case, the shoes care device 1 according to one embodiment of the present invention may be further provided with a dedicated door of the machine room 50 to open and close the machine room 50 separately from the door 30.

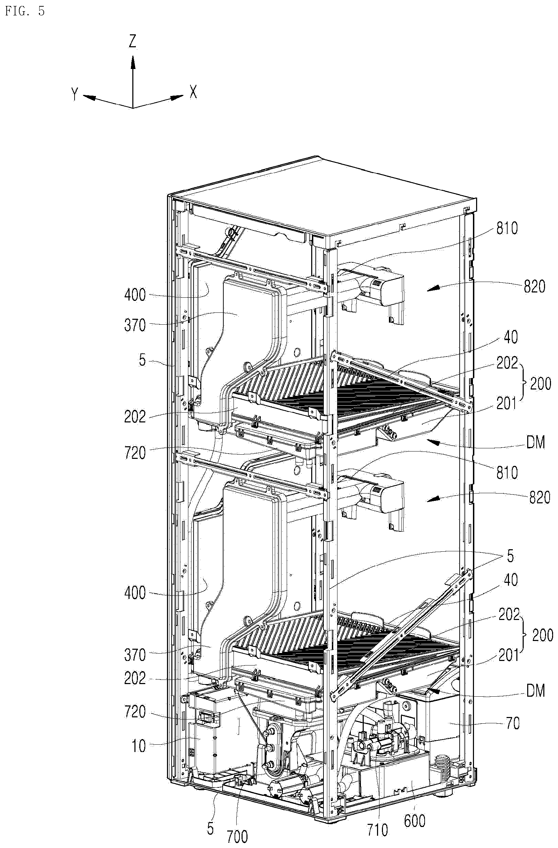

FIG. 5 is a perspective view illustrating a state in which the door 30, the outer cabinet 20, and the inner cabinet 100 are removed from the shoes care device 1 according to one embodiment of the present invention.

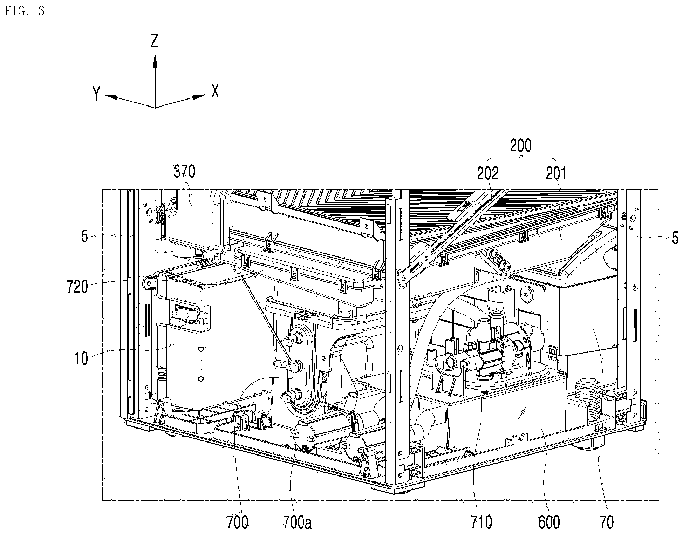

FIG. 6 is a perspective view illustrating a machine room part of the shoes care device 1 illustrated in FIG. 5.

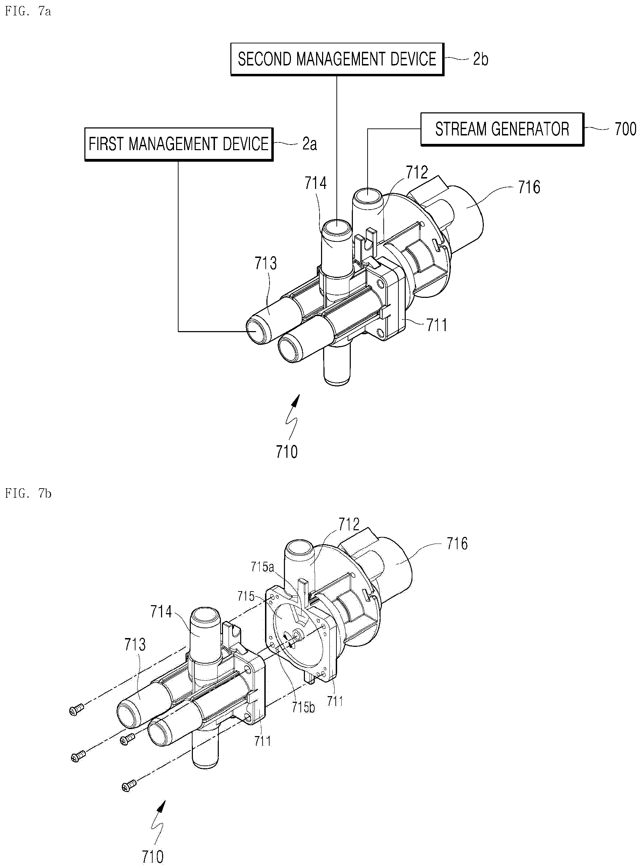

FIG. 7a is a view illustrating the steam valve 710 according to one embodiment of the present invention, and illustrating a connection relationship considering the movement of steam. FIG. 7b is an exploded perspective view illustrating the steam valve illustrated in FIG. 7a.

The shoes care device 1 is provided with the steam generator 700 as a device configured so as to generate moisture inside the inner cabinet 100. The steam generator 700 may be provided inside the machine room 50. The steam generator 700 is configured to generate steam and selectively supply moisture and steam to the inside of the inner cabinet 100.

The shoes care device 1 according to one embodiment may be provided with one steam generator 700, and the shoes care device 1 according to another embodiment may be provided with two or more steam generators 700.

When the shoes care device 1 is provided with one steam generator 700, the steam generator 700 may be configured to supply steam into the inner cabinet 100 of the first management device 2a and/or into the inner cabinet 100 of the second management device 2b.

When the shoes care device 1, one steam generator 700 may be provided with two or more steam generators 700, one of the steam generators 700 may supply steam to the inner cabinet 100 of the first management device 2a, and the other steam generator 700 may supply steam to the inner cabinet 100 of the second management device 2b.

Moist air formed by the steam generator 700 ('air' described in one embodiment of the present invention may be 'air including moisture') is supplied toward the accommodation space 101 of the inner cabinet 100, and moisture may be circulated in the accommodation space 101 of the inner cabinet 100, and accordingly, the moisture may be supplied to the shoes S.

The shoes care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam generator 700 may supply steam to the accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam generator 700 is provided with a separate heater 700a that heats an inner space and water in the inner space and is configured to heat water to generate steam and supply the heated water to the accommodation space 101 of the inner cabinet 100.

An external faucet, etc., as a water supply source for supplying water to the steam generator 700, may be used, or a container-type water supply tank provided on one side of the machine room 50 may be used. The steam generator 700 may generate steam by receiving water from the water supply tank 60.

The water supply tank 60 for the movement of water and the steam generator 700 may be connected by a pipe, a hose, etc.

The steam generator 700 for the movement of steam and the inner cabinet 100 may be connected by a pipe, a hose, etc. In the shoes care device 1 according to one embodiment of the present invention, as described below, the steam generated by the steam generator 700 may be supplied into the inner cabinet 100 after passing through the steam valve 710 and a steam separator 720. In this case, in order to move the steam, the steam generator 700 and the steam valve 710 may be connected by a pipe, a hose, etc., and the steam valve 710 and the steam separator 720 may also be connected by a pipe, a hose, etc.

The steam valve 710 may be disposed adjacent to the steam generator 700, and the steam valve 710 may be provided in the machine room 50. The steam generator 700 and the steam valve 710 may be provided below the second management device 2b.

The steam valve 710 is configured to selectively communicate with each of the accommodation space 101 of the first management device 2a and the accommodation space 101 of the second management device 2b, respectively.

When the steam of the steam generator 700 is supplied to the accommodation space 101 of the first management device 2a and/or the accommodation space 101 of the second management device 2b, the steam valve 710 operates to control whether the stream is supplied or not, and an operation of the stream 710 is driven by the controller 10.

The steam valve 710 includes a valve housing 711, a valve inlet 712, a first valve outlet 713, a second valve outlet 714, a valve disk 715, and a valve motor 716. In the steam valve 710 illustrated in FIGS. 7a and 7b, the other outlets except the valve inlet 712, the first valve outlet 713, and the second valve outlet 714 may be blocked by a stopper and deactivated.

The valve housing 711 forms a body of the steam valve 710, and has a predetermined inner space formed inside.

The valve inlet 712 may have a tubular shape and be coupled to the valve housing 711 to communicate with the inner space of the valve housing 711. The valve inlet 712 is a part connected to the steam generator 700, and steam may flow into the steam valve 710 (inside the valve housing 711) through the valve inlet 712.

The first valve outlet 713 and the second valve outlet 714 may be formed in a tubular shape and be coupled to the valve housing 711 to communicate with the inner space of the valve housing 711.

The first valve outlet 713 and the second valve outlet 714 are outlets through which steam is discharged from the steam valve 710. The first valve outlet 713 is connected to the accommodation space 101 of the first management device 2a, and the second valve outlet 714 is connected to the accommodation space 101 of the second management device 2b.

The valve disk 715 is provided inside the steam valve 710 (inside the valve housing 711) and is configured to open and close a flow path inside the steam valve 710. The valve disk 715 may be configured to selectively open and close a flow path of the first valve outlet 713 and a flow path of the second valve outlet 714.

The valve disk 715 is disposed between the valve inlet 712 and the first valve outlet 713, or between the valve inlet 712 and the second valve outlet 714, inside the valve housing 711. The valve disk 715 allows the valve inlet 712 and the first valve outlet 713 to communicate with each other or block the communication therewith, and also allows the valve inlet 712 and the second valve outlet 714 to communicate with each other or block the communication therewith.

In one embodiment of the present invention, the valve disk 715 may be formed in a circular plate shape and may include a valve hole 715a. The valve hole 715a is a hole penetrating the valve disk 715. The valve disk 715 may be rotatably coupled to the valve housing 711 around a valve rotation axis 715b, and the valve hole 715a may be formed eccentrically from the valve rotation axis 715b.

The valve motor 716 is coupled to the valve housing 711, and the valve motor 716 is coupled to the rotation axis 715b of the valve disk 715 to rotate the valve disk 715.

The controller 10 may control the steam valve 710 by controlling an operation of the valve motor 716.

The valve disk 715 rotates by the operation of the valve motor 716, and the valve disk 715 opens or closes a flow path inside the steam valve 710 (inside the valve housing 711) depending on the degree of rotation of the valve disk 715.

In one embodiment, depending on the degree of rotation of the valve disk 715, when the valve inlet 712 and the first valve outlet 713 communicate with each other through the valve hole 715a, the valve inlet 712 and the second valve outlet 714 are blocked from communicating with each other by the valve disk 715, or when the valve inlet 712 and the second valve outlet 714 communicate with each other through the valve hole 715a, the valve outlet 712 and the first valve outlet 713 may be blocked from communicating with each other by the valve disk 715.

In another embodiment, depending on the degree of rotation of the valve disk 715, the valve inlet 712 may communicate with both the first valve outlet 713 and the second valve outlet 714, or the valve inlet 712 may be blocked from communicating with both the first valve outlet 713 and the second valve outlet 714.

In the shoes care device 1 according to one embodiment of the present invention, the valve disk 715 may close a flow path of the second valve outlet 714 while opening a flow path of the first valve outlet 713, and may also close the flow path of the first valve outlet 713 and open the flow path of the second valve outlet 714.

The steam valve 710 made as described above may operate such that only the valve inlet 712 and the first valve outlet 713 communicate with each other, or only the valve inlet 712 and the second valve outlet 714 communicate with each other.

In the arrangement explained above, all the steam generated by the steam generator 700 may be supplied to the accommodation space 101 of the first management device 2a, or may be supplied to the accommodation space 101 of the second management device 2b. In this case, the steam generated by the steam generator 700 may be supplied to the accommodation space 101 of the first management device 2a or the accommodation space 101 of the second management device 2b without a pressure drop, and even when only one steam generator 700 is provided in the shoes care device 1, the steam may be sufficiently and stably supplied to the accommodation spaces 101 of each of the two inner cabinets 100.

In one embodiment of the present invention, the controller 10 may control the stream valve 710 such that when the heating part 320 of the first management device 2a is turned off (when a heater 321 of the heating part 320 is turned off), the valve disk 715 closes or opens the first valve outlet 713, and when the heating part 320 of the first management device 2a is turned on (when the heater 321 of the heating part 320 is turned on), the valve disk 715 closes the first valve outlet 713.

In addition, the controller 10 may control the stream valve 170 such that when the heating part 320 of the second management device 2b is turned off (when the heater 321 of the heating part 320 is turned off), the valve disk 715 closes or opens the second valve outlet 714, and when the heating part 320 of the second management device 2b is turned on (when the heater 321 of the heating part 320 is turned on), the valve disk 715 closes the second valve outlet 714.

In the shoes care device 1 according to one embodiment of the present invention, by controlling the steam valve 710 by the controller 10, the steam generated in the steam generator 700 may be selectively or simultaneously supplied to the accommodation space 101 of the first management device 2a and the accommodation space 101 of the second management device 2b, and whether the steam is supplied or not may be controlled according to a use state of the shoes care device 1.

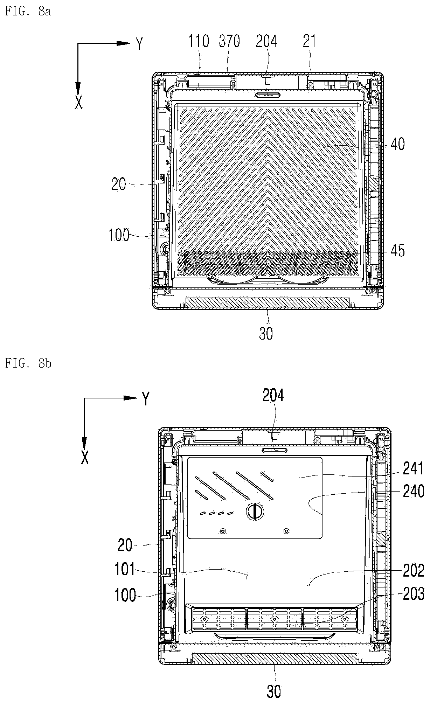

FIG. 8a is a cross-sectional view taken along line D-D' of the shoes care device 1 illustrated in FIG. 3.

FIG. 8b is a view illustrating a state in which the main shelf 40 is removed from the shoes care device 1 illustrated in FIG. 8a.

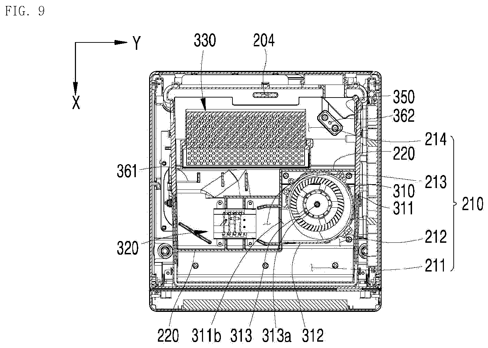

FIG. 9 is a cross-sectional view taken along line E-E' of the shoes care device 1 illustrated in FIG. 3.

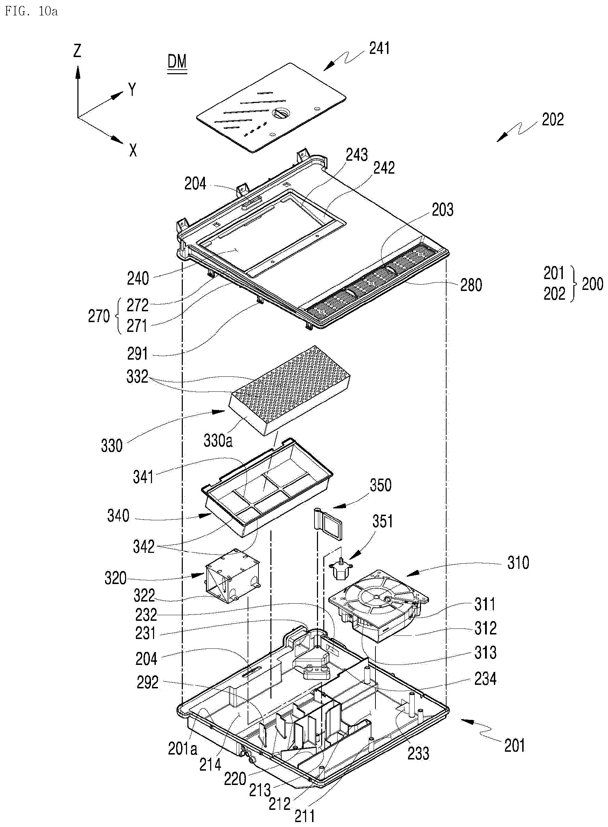

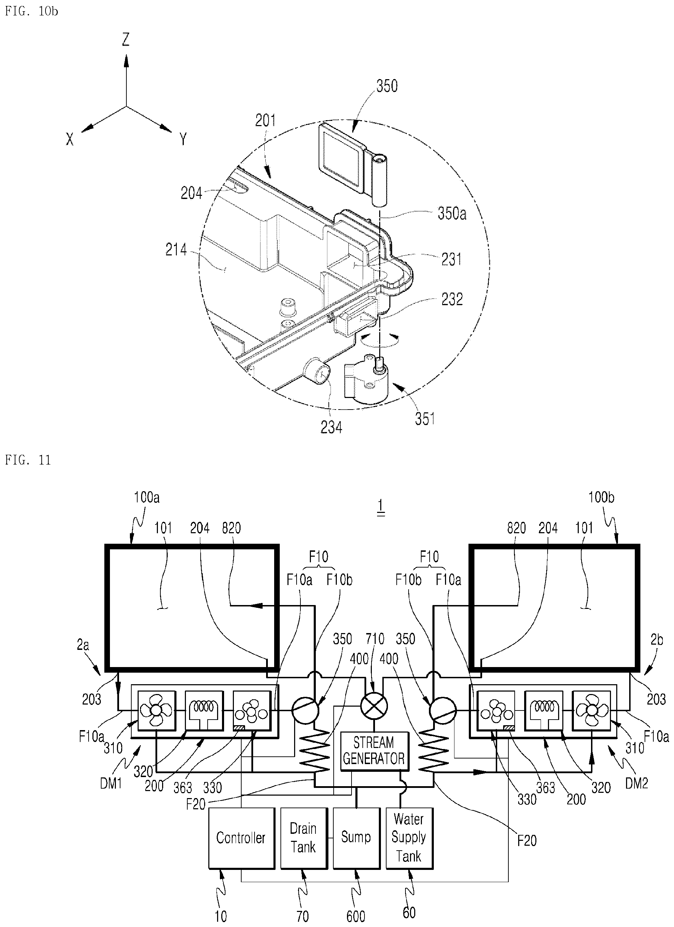

FIG. 10a is an exploded perspective view illustrating a drying module in the shoes care device 1 according to one embodiment of the present invention. FIG. 10b is an exploded perspective view illustrating a partial configuration of the drying module at a part to which the damper 350 is coupled.

In the shoes care device 1 according to one embodiment of the present invention, the dehumidifying part 330 may be used as a means that dehumidifies air

As described above, the dehumidifying part 330 may be provided inside the module housing 200.

The dehumidifying part 330 is configured to have a predetermined volume. The dehumidifying part 330 may be configured to be porous by itself. A plurality of pores may be formed over an entire volume of the dehumidifying part 330, and air may move by penetrating the dehumidifying part 330 through such pores.

When the dehumidifying part 330 is composed of a combination of a plurality of dehumidifying materials, the plurality of dehumidifying materials may be fixed to each other by a separate fixing means, or may be fixed to each other by adhesion.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material 331 according to one embodiment of the present invention may be referred to as a desiccant or an adsorbent.

The dehumidifying material 331 according to one embodiment of the present invention may be formed of a microporous material. The dehumidifying material 331 according to one embodiment of the present invention may include silica gel, activated carbon, activated alumina (AL2O3), diatomaceous earth, etc.

Specifically, the dehumidifying material 331 according to one embodiment of the present invention may be formed of zeolite or may be configured to include zeolite.

The zeolite is a natural and synthetic silicate mineral in which tunnels or open channels having a size of approximately 3 to 10 angstroms (Å) are regularly arranged, and may function as dehumidification by adsorbing the moisture in the air

When the zeolite is heated, moisture adsorbed on the zeolite may be separated into a large amount of steam. According to the characteristics of the zeolite, the zeolite may be regenerated in a state capable of not only performing the dehumidification function to remove the moisture from the air but also performing the dehumidification function by heating the zeolite and separating the moisture adsorbed to the zeolite.

The zeolite may be formed in the form of small grains (or stones) having the size (diameter) of several micrometers to several tens of micrometers, and the dehumidifying material 331 described in one embodiment of the present invention may refer to a combination of the grains (or stones). Each of the grains (or stones) may be agglomerated or combined with each other to form a single structure.

In another embodiment, the dehumidifying part 330 may include a dehumidifying body 330a and the dehumidifying material 331.

The dehumidifying body 330a may be formed to have a predetermined volume. In one embodiment, the dehumidifying body 330a may be formed in a substantially hexahedral shape.

In order to allow air to move through the dehumidifying body 330a, the dehumidifying body 330a may be provided with a plurality of dehumidification through holes 332 penetrated in one direction. A cross section of the dehumidifying through hole 332 may be formed in a circular shape, a polygonal shape, etc. The dehumidifying through hole 332 may have a hexagonal cross section.

In the dehumidifying body 330a, the dehumidifying through hole 332 may all have the same shape and size, or may have different shapes and sizes.

The dehumidifying body 330a may be formed of or include materials such as synthetic resin, metal, ceramic, etc. The dehumidifying body 330a may be formed of a combination of fibers, and may be formed of a nonwoven fabric, etc.

The dehumidifying material 331 may be coated on the dehumidifying body 330a. The dehumidifying material 331 may be coated on the outside and inside of the dehumidifying body 330a. Specifically, the dehumidifying material 331 may be coated on a surface in which the dehumidifying through hole 332 is formed.

When the dehumidifying body 330a is formed of a combination of fibers, the zeolite may be first coated on each fiber as the dehumidifying material 331, and the zeolite-coated fiber may be processed to form the dehumidifying body 330a and simultaneously form the dehumidifying part 330.

Regarding the coating of the zeolite, a manufacturing method of a zeolite coated ceramic paper (Korean Patent No. 10-1004826) is known, and Korean Patent No. 10-1173213 and Korean Patent No. 10-0941521 also describes a method of coating zeolite on a surface of a material. The dehumidifying part 330 according to one embodiment of the present invention may be formed by coating the gelled zeolite precursor on the dehumidifying body 330a or materials constituting the dehumidifying body 330a and then performing heat treatment when the dehumidifying material 331 is formed of the zeolite.

In one embodiment of the present invention, the coating of the dehumidifying material 331 (zeolite) may be performed by using various known or possible methods, and is not limited to a certain manufacturing method related to the coating of the dehumidifying material 331.

The blowing part 310 is provided inside the connection path F10. A blowing fan 313 may be provided inside the blowing part 310. Since the blowing part 310 is provided in the connection path F10, air flows in the connection path F10 when the blowing part 310 is driven, and since the connection path F10 also communicates with the accommodation space 101 of the inner cabinet 100, the air flows and moves in the accommodation space 101 by driving the blowing part 310.

In this way, the air may be sucked from the inner cabinet 100 into the connection path F10 by the driving of the blowing part 310 (a rotation of the blowing fan 313), and the air inside the connection path F10 may be ventilated.

In one embodiment, the blowing part 310 is provided inside the module housing 200 forming the connection path F10, and the blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the shoes care device 1 by the driving of the blowing part 310.

Dry air may be supplied to the inside of the inner cabinet 100 by the blowing part 310.

The heating part 320 is provided at one side of the dehumidifying part 330 and the blowing part 310 in the connection path F10. The heating part 320 may be provided inside the module housing 200. Based on a movement direction of the air inside the module housing 200, the module housing 200 may be arranged in an order of the blowing part 310, the heating part 320, and the dehumidifying part 330. That is, the air introduced into the outlet 203 of the module housing 200 moves along the connection path F10 by passing sequentially through the blowing part 310, the heating part 320, and the dehumidifying part 330.

The heating part 320 is disposed inside the module housing 200 and is configured to heat the air of the module chamber 210 inside the module housing 200.

The heating part 320 may be configured to heat the dehumidifying part 330. The heating part 320 may be configured to heat the dehumidifying material 331 constituting the dehumidifying part 330.

The air heated by the heating part 320 by the driving of the blowing part 310 moves directly to the dehumidifying part 330, thus heating the dehumidifying part 330. To this end, the heating part 320 is disposed inside the module housing 200 adjacent to the dehumidifying part 330. Specifically, the heating part 320 is disposed inside the module housing 200 adjacent to the dehumidifying part 330 based on a movement path of the air inside the module housing 200.

In the module housing 200, the dehumidification by the dehumidifying material 331 or the regeneration of the dehumidifying material 331 may be achieved by selectively heating the heating part 320.

The heating part 320 may be fixedly coupled to the module housing 200 in the module housing 200.

The heating part 320 may be made up of various devices and structures within a range capable of heating the air inside the module housing 200 or supplying heat to the dehumidifying part 330.

The heating part 320 may be formed of an electric heater 321. In one embodiment of the present invention, the heating part 320 may include the heater 321. The heater 321 includes a heating element, and may be configured to supply heat to the periphery while the heating element generates heat by supplied electric energy. The heater 321 may include a nichrome wire as the heating element.

The heater 321 of the heating part 320 may be formed in a ring shape, and the air may move by penetrating the center and surroundings of the ring-shaped heater 321 and be simultaneously heated. The heater 321 of the heating part 320 may be repeatedly formed in a second module chamber 213 along the movement direction of air.

The heater 321 of the heating part 320 may be formed in a circular ring shape or a rectangular ring shape.

The heating part 320 may include a heater flange 322 to which the heater 321 is fixed

The heater flange 322 may be formed in the form of a metallic plate.

The heater flange 322 may be formed of a combination of flat plates in the second module chamber 213 along the movement direction of air. The heater flange 322 has a cross section that may be formed of a plate shape or a combination of plates in the second module chamber 213 along the movement direction of air (the second direction (Y direction)).

The heater flange 322 may include an outer flange 322a and an inner flange 322b.

The outer flange 322a may be formed in a tubular shape along the second direction (Y direction). An interior of the outer flange 322a is provided with a space to move air along the movement direction of air (a direction parallel to the second direction (Y direction)) in the second module chamber 213.

The inner flange 322b is fixed to the interior of the outer flange 322a. The inner flange 322b may include two or more plates crossing each other, and the heater 321 of the heating part 320 may be fixed to the inner flange 322b.

The heater flange 322 may be formed in various forms that fix the heater 321 of the heating part 320 and do not interfere with a flow of air moving through the second module chamber 213.

In one embodiment of the present invention, the blowing part 310, the heating part 320, the dehumidifying part 330, and the module housing 200 may form one set.

The set may be provided in a plural form. The shoes care device 1 according to one embodiment may be provided with two sets.

Such a set may be provided in each of the first management device 2a and the second management device 2b.

Such a set may form a drying module DM in the shoes care device 1 according to one embodiment of the present invention.

That is, in one embodiment of the present invention, the drying module DM may include the module housing 200, the blowing part 310, the heating part 320, and the dehumidifying part 330. Furthermore, the drying module DM is provided in each of the first management device 2a and the second management device 2b.

In the shoes care device 1 according to one embodiment of the present invention, a plurality of drying modules DM may be provided.

In the shoes care device 1 according to one embodiment of the present invention, a pair of drying modules DM may be provided. When the shoes care device 1 is provided with the pair of drying modules DM, one of the drying modules DM may form a 'drying module A (DM1)' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the shoes care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The module housing 200 may be fixedly coupled to a lower side of the inner cabinet 100. The module housing 200 may be detachably coupled to a lower side of the inner cabinet 100.

The module housing 200 includes the module chamber 210 that is a space having other components accommodated inside. That is, the module chamber 210 is a space inside the module housing 200 distinguished from an external space of the module housing 200. As described above, the module housing 200 forms part of the connection path F10, and accordingly, the module chamber 210 is configured to communicate with a space outside the module housing 200. The module chamber 210 communicates with the accommodation space 101 of the inner cabinet 100.

The module housing 200 may include a module case 201 and a module cover 202.

The module case 201 and the module cover 202 may be formed by injection molding, respectively, and may be assembled with each other after manufacturing to form the module housing 200.

The module case 201 is formed in the form of a container that is concave substantially downwards, and forms the module chamber 210 of the module housing 200.

The module case 201 may be configured in the form of a container opened upwards, and includes a module opening 201a.

In a plan view, an area of the module opening 201a may be larger than or equal to an area of the module chamber 210.

The module chamber 210 may include a first module chamber 212, a second module chamber 213, and a third module chamber 214. The module chamber 210 may include a suction module chamber 211.

In order to distinguish between the suction module chamber 211, the first module chamber 212, the second module chamber 213, and the third module chamber 214, a module partition wall 220 may be formed inside the module housing 200. Furthermore, the module partition wall 220 guides the movement of air so that air moves in a predetermined direction inside the module housing 200.

The suction module chamber 211 is a first space where air is introduced into the module housing 200.

The first module chamber 212 is a space in which the blowing part 310 is accommodated, the second module chamber 213 is a space in which the heating part 320 is accommodated, and the third module chamber 214 is a space in which the dehumidifying part 330 is accommodated.

In one embodiment of the present invention, the suction module chamber 211, the first module chamber 212, the second module chamber 213, and the third module chamber 214 may be formed at different positions in a plan view.

In addition, the air of the module chamber 210 may be configured to move the suction module chamber 211, the first module chamber 212, the second module chamber 213, and the third module chamber 214 sequentially. That is, when the blowing part 310 is driven, air moves sequentially through the suction module chamber 211, the first module chamber 212, the second module chamber 213, and the third module chamber 214 inside the module housing 200.

The module case 201 may include a dry air outlet 231 and a wet air outlet 232.

The dry air outlet 231 may be formed in a hole shape opened to allow air in the third module chamber 214 to flow out. The dry air outlet 231 is formed adjacent to the third module chamber 214. The dry air outlet 231 may be formed on one edge of the module case 201. Furthermore, the dry air outlet 231 may be connected to the accommodation space 101 through the dry air duct 370 and the nozzle duct 810.

The wet air outlet 232 may be formed in a hole shape opened to allow the air in the third module chamber 214 to flow out. The wet air outlet 232 is formed adjacent to the third module chamber 214. The wet air outlet 232 may be formed on a frame on one side of the module case 201. Furthermore, the wet air outlet 232 may be connected to the condenser 400.

The dry air outlet 231 and the wet air outlet 232 may be formed adjacent to each other. The dry air outlet 231 and the wet air outlet 232 may be formed adjacent to any one vertex part of the module housing 200.

The suction module chamber 211 is formed adjacent to the first module chamber 212, and a bottom surface of the suction module chamber 211 may be inclined downwardly toward the first module chamber 212. Accordingly, air introduced into the suction module chamber 211 may naturally move toward the first module chamber 212 by hitting the bottom surface of the suction module chamber 211 forming an inclined surface, and a condensed water introduced into the suction module chamber 211 may move along the bottom surface of the suction module chamber 211 forming the inclined surface, and may move to the first module chamber 212.

The blowing part 310 may be assembled to the module housing 200 while being spaced apart from a bottom surface of the first module chamber 212. Furthermore, in this case, the blowing part 310 may be configured such that air may be introduced from a lower side of the first module chamber 212 to an interior of the blowing part 310 inside the first module chamber 212.

The module case 201 may include a first condensed water discharge hole 233.

The first condensed water discharge hole 233 is formed in a hole shape penetrating the module case 201. The first condensed water discharge hole 233 is formed on an edge of the module case 201 adjacent to a condenser 400 and formed to be equal to or lower than the bottom surface of the first module chamber 212, and communicates with the condenser 400. Among the bottom surfaces of the first module chamber 212, the first condensed water discharge hole 233 may form the lowest part, or the bottom surface of the first module chamber 212 may be formed such that a height thereof is lowered toward or at least equal to the first condensed water discharge hole 233.

As such, the first condensed water discharge hole 233 may be lower than the bottom surface of the first module chamber 212, and accordingly, the condensed water introduced into the first condensed water discharge hole 232 may move toward the first condensed water discharge hole 233 and flow into the condenser 400 through the first condensed water discharge hole 233.

Meanwhile, since the first condensed water discharge hole 233 is a hole in which the module housing 200 and the condenser 400 communicate with each other, the air inside the condenser 400 may flow into the module housing 200 through the first condensed water discharge hole 233. The air introduced into the module housing 200 through the first condensed water discharge hole 233 from an interior of the condenser 400 may move along the first module chamber 212, the second module chamber 213, and the third module chamber 214 by an operation of the blowing part 310 and may be introduced again into the condenser 400 and condensed.

The shoes care device 1 according to one embodiment of the present invention includes the condenser 400 coupled to an outer surface of the inner cabinet 100 and forming a regeneration path F20. In a plan view, the first module chamber 212 may be provided between the second module chamber 213 and the condenser 400. Since the first module chamber 212 is disposed between the second module chamber 213 and the condenser 400, a direct heat exchange between the condenser 400 and the heating part 320 is blocked, and the heat may be prevented from being transferred to the condenser 400 when the heating part 320 is heated inside the second module chamber 213.

Accordingly, when the dehumidifying part 330 is regenerated, condensation depending on heating of air by the heater 321 of the heating part 320 and cooling of air inside the condenser 400 may be effective performed.

The dehumidifying part 330 may be coupled to the module housing 200 while being spaced apart from a bottom surface of the third module chamber 214. Furthermore, in this case, the air inside the third module chamber 214 may move downwards through the dehumidifying part 330 from an upper side of the third module chamber 214.

The module case 201 may include a second condensed water discharge hole 234.

The second condensed water discharge hole 234 is formed in a hole shape penetrating the module case 201. The second condensed water discharge hole 234 is formed on the edge of the module case 201 adjacent to the condenser 400 and formed to be equal to or lower than the bottom surface of the third module chamber 214, and communicates with the condenser 400. Among the bottom surfaces of the third module chamber 214, the second condensed water discharge hole 234 may form the lowest part, or the bottom surface of the third module chamber 214 may be formed such that a height thereof is lowered toward or at least equal to the second condensed water discharge hole 234.

The second condensed water discharge hole 234 may be formed adjacent to the wet air outlet 232.

In this way, the second condensed water discharge hole 234 may be lower than the bottom surface of the third module chamber 214, and accordingly, condensed water introduced into the third module chamber 214 may move toward the second condensed water discharge hole 234, and may flow into the condenser 400 through the second condensed water discharge hole 234.

Meanwhile, since the second condensed water discharge hole 234 is a hole in which the module housing 200 and the condenser 400 communicate with each other, the air inside the condenser 400 may flow into the module housing 200 through the second condensed water discharge hole 234. In this way, the air introduced from the interior of the condenser 400 into the module housing 200 through the second condensed water discharge hole 234 moves directly to the wet air outlet 232 by the driving of the blowing part 310, and may be introduced again into the condenser 400 and condensed.

The module housing 200 may include the module cover 202.

The module cover 202 is coupled to the module case 201 while shielding the module opening 201a from an upper side of the module case 201. The module cover 202 may be detachably coupled to the module case 201. A plurality of locking projections 292 may protrude from one of the module cover 202 and the module case 201, and a plurality of locking grooves 291 into which the locking projections 292 are inserted and locked may be formed on the other. The locking projections 292 and locking grooves 291 are provided in a plural form, respectively, and may be spaced apart along an edge of the module housing 200 and repeatedly formed.

With the blowing part 310, the heating part 320, and the dehumidifying part 330 accommodated in the module case 201, the module cover 202 may shield the blowing part 310, the heating part 320, and the dehumidifying part 330 and may be coupled to the module case 201.

The shoes care device according to one embodiment of the present invention may be formed in a structure in which the dehumidifying part 330 may be detachable from the module housing 200. The structure of the shoes care device provides an advantageous advantage in maintaining and managing the dehumidifying part 330 and the shoes care device 1 on the whole.

On the other hand, the dehumidifying part 330 may be repeatedly used by regeneration, but with the repeated use, the dehumidifying part 330 needs to be replaced.

Considering these descriptions, the shoes care device 1 according to one specific embodiment of the present invention may be configured to separate and replace the dehumidifying part 330.

In one embodiment of the present invention, the module cover 202 of the module housing 200 may form a bottom surface of the inner cabinet 100.

The module cover 202 may form a boundary surface between the inner cabinet 100 and the module housing 200. The module cover 202 may be formed in a substantially rectangular shape.

The module cover 202 may be configured in substantially parallel with the horizontal direction.

Alternatively, the module cover 202 may be inclined to any one side. In one embodiment, an upper surface of the module cover 202 may be inclined downwardly toward the first direction (X direction) (a front side of the shoes care device 1).

In one embodiment of the present invention, the main shelf 40 is mounted in close contact with an upper side surface of the module cover 202, and the main shelf 40 mounted on the upper side surface of the module cover 202 is also configured to be inclined when the upper side surface of the module cover 202 is inclined. In this case, since an upper surface of the main shelf 40 is inclined, water (e.g., condensed water) placed on the upper surface of the main shelf 40 may flow along an inclined direction.

The shoes care device 1 may include a dehumidifying material cover 241.

The dehumidifying material cover 241 forms part of the module cover 202 that is the bottom of the inner cabinet 100. Furthermore, the dehumidifying material cover 241 may be detached from the module cover 202 of the inner cabinet 100 or may be hinge-coupled to the module cover 202.

In the module cover 202, a dehumidifying material exit 240 which is an opening of a shape and a size corresponding to the dehumidifying material cover 241 may be formed. The dehumidifying material cover 241 may be configured to open and close the dehumidifying material exit 240. The dehumidifying material cover 241 may be tightly coupled to the dehumidifying material exit 240. At least a part of the dehumidifying material cover 241 may be separated from the module cover 202. In one embodiment, the dehumidifying material exit 240 of the module cover 202 may be opened while completely separating the dehumidifying material cover 241 from the module cover 202, and in another embodiment, the dehumidifying material cover 241 of the module cover 202 may be opened while rotating the dehumidifying material cover 241 around a hinge axis. The dehumidifying part 330 may be introduced into or withdrawn from the module housing 200 through the dehumidifying material exit 240.

The dehumidifying material exit 240 and the dehumidifying material cover 241 may be formed in a position corresponding to the third module chamber 214 in a plan view. That is, the dehumidifying material exit 240 and the dehumidifying material cover 241 may be formed directly above the third module chamber 214. The shoes care device 1 according to one embodiment of the present invention may be configured such that the first module chamber 212 and the second module chamber 213 are not exposed in a plan view in a state where the dehumidifier cover 241 is opened.

When the dehumidifying material cover 241 is opened in the module cover 202, the third module chamber 214 disposed on a lower part of the module cover 202 is exposed through the dehumidifying material exit 240 of the module cover 202, and the dehumidifying part 330 may be settled inside the module case 201, or may be immediately withdrawn and separated from the module case 201.

The sizes and shapes of the dehumidifying material cover 241 and the dehumidifying material exit 240 are variously provided within the range capable of withdrawing or inserting the dehumidifying part 330.

The dehumidifying material cover 241 may be formed in a rectangular plate shape.

The length of the dehumidifying material cover 241 in the first direction (X direction) may be equal to or longer than the length of the dehumidifying part 330, and the length of the dehumidifying material cover 241 in the second direction (Y direction) may be equal to or longer than the length of the dehumidifying part 330.

As described above, in the shoes care device 1 according to one embodiment of the present invention, the heating part 320 and the dehumidifying part 330 are formed at different positions in a top plane view, and when the dehumidifying material cover 241 is opened on the module cover 202 that forms the bottom surface of the inner cabinet 100, the dehumidifying part 330 disposed directly below the dehumidifying material cover 241 may be withdrawn from the module housing 200, and the dehumidifying part 330 may be easily replaced by the user

In addition, since only the third module chamber 214 is exposed in a state in which the dehumidifier cover 241 is opened, and the first module chamber 212 and the second module chamber 213 are not exposed, the blowing part 310 accommodated in the first module chamber 212 and the heating part 320 accommodated in the second module chamber 213 are not exposed. That is, since the blowing part 310 and the heating part 320 are not directly exposed to the user, safety accidents due to an unintended operation of the blowing part 310 and/or the heating part 320 can be prevented.

The dehumidifying material cover 241 may be configured to separately shield the dehumidifying part 330. A space between the dehumidifying material cover 241 and the dehumidifying part 330 may form a part of the connection path F10.

As described above, the outlet 203 forms an inlet through which the air inside the inner cabinet 100 is sucked into the module housing 200. The outlet 203 may form a start part of the connection path F10. The outlet 203 may be formed in the shape of a hole vertically penetrated from the bottom surface (the upper surface of the module cover 202) of the inner cabinet 100.

A network such as a grid shape, a mesh shape, etc., may be formed on the outlet 203.

The outlet 203 may be formed parallel to the second direction (Y direction). That is, the outlet 203 may be formed in a long hole shape in the module cover 202 along the second direction (Y direction).

The outlet 203 may be formed on an edge of the module cover 202. The outlet 203 may be formed on the edge of the module cover 202 along the second direction (Y direction).

The outlet 203 may be formed on a front part or a rear part of the module cover 202 based on the first direction (X direction).

The outlet 203 may be disposed relatively close to the door 30 in the module cover 202. That is, the outlet 203 may be disposed relatively in the front of the module cover 202.

The upper surface of the module cover 202 may be inclined downwardly toward the outlet 203. That is, a part of the module cover 202 where the outlet 203 is formed may be configured to be the lowest. Accordingly, when water is present on the module cover 202 or the main shelf 40, such water may flow along the surface of the module cover 202 by gravity and flow into the inlet 203.

In the shoes care device 1 according to one embodiment of the present invention, the module chamber 210 is provided inside the module housing 200, and the module chamber 210 includes a first module chamber 212, a second module chamber 213, and the third module chamber 214. The first module chamber 212, the second module chamber 213, and the third module chamber 214 may be formed at different positions in a plan view. That is, the blowing part 310, the heating part 320, and the dehumidifying part 330 may be disposed at different positions in the module housing 200. According to one embodiment of the present invention, the blowing part 310, the heating part 320, and the dehumidifying part 330, which are main means for drying the air inside the inner cabinet 100 and main means for regenerating the dehumidifying part 330, are disposed together in the module chamber 210 of the module housing 200. Accordingly, the blowing part 310, the heating part 320, and the dehumidifying part 330 are disposed at positions considerably close to each other.

In one embodiment of the present invention, the module case 201 of the module housing 200 may be integrally formed by injection molding. In this case, the bottom parts of the module housing 200 may be integrally formed, the bottom parts may not be assembled with each other, and no gaps may be formed in the bottom parts.

In the arrangement explained above, the condensed water can be effectively prevented from leaking from the module housing 200. In addition, a vertical height of the module housing 200 can be minimized.

When moisture remains at an unintended part inside the shoes care device 1, such moisture may reproduce bacteria or cause odors. This is why there is need for countermeasures to solve the problems, and the shoes care device 1 according to one embodiment of the present invention can effectively prevent water from leaking in consideration of such problems.

In the shoes care device 1 according to one embodiment of the present invention, the air in the module chamber 210 may sequentially move the first module chamber 212, the second module chamber 213, and the third module chamber 214. Accordingly, since the third module chamber 214 and a drying flow path F10b may be connected in the shortest distance, which provides excellent drying efficiency by the dehumidifying part 330, and air heated by the heating part 320 moves directly to the dehumidifying part 330 to form the shoes care device 1 with excellent regeneration efficiency.

In the shoes care device 1 according to one embodiment of the present invention, the module housing 200 includes the suction module chamber 211, and the bottom surface of the suction module chamber 211 may be inclined downwardly toward the first module chamber 212. Accordingly, the air introduced through the outlet 203 moves naturally to the first module chamber 212 by hitting the bottom surface of the suction module chamber 211, and the condensed water introduced into the outlet 203 moves to the first module chamber 212 such that the condensed water can be easily drained.

The shoes care device 1 according to one embodiment of the present invention may include the condenser 400, and the module case 201 may include the first condensed water discharge hole 233. In addition, the module case 201 may include the second condensed water discharge hole 234. Accordingly, the dehumidifying part 330 may be effectively regenerated, and condensed water inside the module housing 200 may be easily discharged to the condenser 400.

In the shoes care device 1 according to one embodiment of the present invention, steam generated by the steam generator 700 is supplied to the accommodation space 101 of the inner cabinet 100, and to this end, the shoes care device 1 includes a steam inlet 204.

The steam inlet 204 forms an inlet through which steam is supplied to the accommodation space 101 of the inner cabinet 100.

In the shoes care device according to one embodiment of the present invention, the steam inlet 204 is formed in the module housing 200.

The steam inlet 204 may be formed in a rear part of the module housing 200 based on the first direction (X direction). The steam inlet 204 may be formed to vertically penetrate the module housing 200. The steam inlet 204 may be formed to vertically penetrate the module case 201 and the module cover 202. The steam inlet 204 may be formed in the center in a right-left direction at the rear part of the module housing 200.

The steam inlet 204 may be formed on a rear edge of the module housing 200, and may be formed directly behind a position where the third module chamber 214 is formed. The third module chamber 214 and the steam inlet 204 are shielded from each other.

In the module housing 200, the module cover 202 forms the bottom surface of the accommodation space 101, and when the module housing 200 is coupled to the inner cabinet 100, the steam inlet 204 is formed behind the bottom of the inner cabinet 100.

The steam generator 700 and the steam valve 710 are disposed in a lower part, the distance from the steam generator 710 to the steam inlet 204 can be reduced by forming the module housing 200, and the steam inlet 204 in a rear part of the module housing 200, and an increase in the load required for the supply of steam can be prevented. Accordingly, steam may be smoothly supplied from the steam generator 700 to the steam inlet 204

FIG. 11 is a diagram illustrating a connection relationship between components and a flow of fluid in the shoes care device 1 according to one embodiment of the present invention.

The connection path F10 forms a movement path of air connected from the outlet 203 to the nozzle 820. That is, the outlet 203 may form an inlet of the connection path F10, and the nozzle 820 may form an outlet of the connection path F10.

The outlet 203 may be coupled to communicate with the inner cabinet 100, and the nozzle 820 may be provided inside the inner cabinet 100. Except the outlet 203 and the nozzle 820, one part of the connection path F10 may be provided inside the inner cabinet 100, and the other part may be provided outside the inner cabinet 100.

The air inside the inner cabinet 100 moves to the connection path F10 through the outlet 203, and the air passing through the connection path F10 moves back into the inner cabinet 100 through the nozzle 820. As such air flow is repeated, the air circulation is performed in the shoes care device 1.

In the nozzle 820, a hole through which air is discharged is formed in the accommodation space 101 of the inner cabinet 100, and the nozzle 820 may form a last part of the connection path F10.

In the shoes care device 1 according to one embodiment of the present invention, since the nozzle 820 is configured to be movable to various positions inside the inner cabinet 100, the shoes may be managed in various positions.

As described above, the dehumidifying part 330 is disposed in the connection path F10. The air moving through the connection path F10 passes through the dehumidifying part 330, and the dehumidifying part 330 absorbs moisture from the air moving through the connection path F10 such that the air from which moisture has been removed may be supplied into the inner cabinet 100.

The connection path F10 may be divided into a conversion flow path FlOa and a drying flow path F10b. The conversion flow path F10a and the drying flow path F10b form a movement path of air sequentially connected to each other. The air in the connection path F10 may sequentially move through the conversion flow path FlOa and the drying flow path FlOb.

The conversion flow path FlOa forms an upstream section of the connection path F10, which is connected to the outlet 203. The conversion flow path FlOa may be a section in which the blowing part 310, the heating part 320, and the dehumidifying part 330 are disposed. The conversion flow path FlOa may be formed by the module housing 200, and the module chamber 210 inside the module housing 200 may form the conversion flow path F10a.

The conversion flow path FlOa may be a section in which humid air moves and dries. The conversion flow path FlOa may be a section in which air is dehumidified by the dehumidifying part 330.

Meanwhile, the conversion flow path FlOa may be a section in which the dehumidifying part 330 (the dehumidifying material 331) are regenerated.

The drying flow path F10b forms a downstream section of the connection path F10, which connects the conversion flow path FlOa to the nozzle 820. A flow path formed by the drying air duct 370, the nozzle duct 810, and the nozzle 820 may form the drying flow path FlOb.

The drying passage F10b may be a section in which dry air with moisture removed therefrom moves.

When the drying module DM operates in the moisture absorption mode, the drying flow path F10b communicates with the conversion flow path F10a, and when the drying module DM operates in the regeneration mode, the drying flow path F10b and the conversion flow path FlOa may not communicate with each other such that the drying flow path F10b and the conversion flow path FlOa block each other

Accordingly, when air is dehumidified by the dehumidifying part 330 in the conversion flow path FlOa, the dried air moves through the drying flow path FlOb.

The dry air duct 370 may be fixedly coupled to an outer wall surface of the inner cabinet 100, and the nozzle duct 810 may be provided inside the inner cabinet 100.

As the dry air duct 370 is tightly coupled to an inner rear plate 110 of the inner cabinet 100, a flow path may be formed between the dry air duct 370 and the inner cabinet 100 (the inner rear plate 110), and such a flow path may form a part of the drying flow path F10b. A lower part of the dry air duct 370 communicates with the dry air outlet 231 of the module housing 200, an upper part thereof communicates with the nozzle duct 810, which connect the interior of the module housing 200 and the interior of the nozzle duct 810 for mutual communication.

As described above, after humid air in the accommodation space 101 of the inner cabinet 100 flows into the conversion flow path FlOa, the air is dehumidified by the dehumidifying part 330 and converted into dry air, and the dry air can be resupplied to the accommodation space 101 of the inner cabinet 100 through the drying flow path F10b.

The regeneration path F20 forms a movement path of a fluid

The regeneration path F20 forms a passage through which air and/or condensed water inside the shoes care device moves

The regeneration path F20 forms a path through which air and/or condensed water passing through the dehumidifying part 330 moves when the dehumidifying material 331 is regenerated. The regeneration path F20 may be entirely or partially formed of a pipe, a hose, a tube, a duct, a housing, or a combination thereof.

Moisture generated during the regeneration process of the dehumidifying material 331 needs to be discharged through a separate flow path separated from the drying flow path F10b, which is a flow path through which dry air moves. Accordingly, the shoes care device 1 according to one embodiment of the present invention includes the regeneration path F20, and when the dehumidifying material 331 is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path F10b of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam generator 700.

In the shoes care device 1 according to one embodiment of the present invention, when the drying module DM operates in the regeneration mode, the regeneration path F20 communicates with the conversion flow path FlOa, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path FlOa, humid air containing moisture separated from the dehumidifying part 330 moves through the regeneration path F20.

In one embodiment of the present invention, the damper 350 may be formed in the form of a damper valve.

The damper 350 may be rotatably coupled to the module housing 200. The damper 350 may be coupled to the module housing 200 in a form accommodated in the module housing 200.

As described above, in the module housing 200, the dry air outlet 231 forming an inlet of the drying flow path F10b is formed as a passage of the connection path F10, and the wet air outlet 232 forming an inlet of the regeneration path F20 is formed.

The damper 350 controls a movement path of air passing through the dehumidifying material 331 in the module housing 200. Depending on the operation of the damper 350, the air passing through the dehumidifying material 331 may move into the inner cabinet 100 through the nozzle 820 or may move into the regeneration path F20.

The damper 350 may be configured to open the regeneration path F20 while blocking the drying flow path F10b, or to open the drying flow path F10b while blocking the regeneration path F20.

The damper 350 may be configured to selectively shield the dry air outlet 231 and the wet air outlet 232. The damper 350 may be configured to selectively seal the dry air outlet 231 and the wet air outlet 232.

The damper 350 may selectively block one of the dry air outlet 231 and the wet air outlet 232. When the damper 350 opens the dry air outlet 231 while blocking the wet air outlet 232, the air passing through the dehumidifying material 331 may move into the inner cabinet 100 through the nozzle 820, and when the damper 350 opens the wet air outlet 232 while blocking the dry air outlet 231, the air passing through the dehumidifying material 331 may be condensed while moving through the regeneration path F20.

In the shoes care device 1 according to one embodiment of the present invention, the damper 350 may be configured to be hinge-rotatable around a hinge axis 350a formed on one side. The hinge axis 350a of the damper 350 may be parallel to the third direction (Z direction). In addition, the shoes care device 1 may include a damper motor 351 configured to rotate the damper 350 around the hinge axis 350a of the damper 350. The damper motor 351 may be formed as an electric motor and may be configured to rotate the damper 350 bidirectionally.

When the damper 350 opens the dry air outlet 231 and seals the wet air outlet 232, the air inside the inner cabinet 100 moves along the connection path F10 and is circulated by sequentially passing through the inlet 203, the module housing 200 (the blowing part 310 and the dehumidifying part 330, the dry air outlet 231, the dry air duct 370, the nozzle duct 810, and the nozzle 820.

When the damper 350 seals the dry air outlet 231 and opens the wet air outlet 232, the air moves along the conversion flow path FlOa and the regeneration path F20 and is circulated by sequentially passing through the module housing 200 (the blowing part 310, the heating part 310, and the dehumidifying part 330), the wet air outlet 232, and the condenser 400.

In one embodiment of the present invention, the controller 10 may control the damper motor 351 such that the damper 350 closes the dry air outlet 231 and opens the wet air outlet 232 when the heating part 320 is turned on. In addition, the controller 10 may control the damper motor 351 such that the damper 350 opens the dry air outlet 231 and closes the wet air outlet 232 when the heating part 320 is turned off.

Accordingly, by controlling the damper motor 351 by the controller 10, the damper 350 may open the drying flow path F10b and close the regeneration path F20 when the heating part 320 is turned off, and may close the drying flow path F10b and open the regeneration path F20 when the heating part 320 is turned on.

The damper motor 351 may be controlled individually in each of the first management device 2a and the second management device 2b.

Referring to FIG. 11, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path FlOa of the first management device 2a may flow through the drying flow path F10b, and the air in the conversion flow path FlOa of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path FlOa of the first management device 2a may flow along the regeneration path F20, and the air in the conversion flow path FlOa of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

In the shoes care device 1 according to one embodiment of the present invention, the first management device 2a and the second management device 2b individually include the inner cabinet 100, the connection path F10, the blowing part 310, and the dehumidifying part 330. In addition, the shoes care device 1 includes the steam generator 700 and the steam valve 710. Accordingly, the degree of the supply of steam, the degree of dehumidification by the dehumidifying part 330, and the flow of air circulated along the connection path F10 may be different in each of the first management device 2a and the second management device 2b, and the first management device 2a and the second management device 2b may manage shoes under different conditions.

In addition, the first management device 2a and the second management device 2b include the module housing 200, the blowing part 310, the heating part 320, the dehumidifying part 330, and the drying flow path F10b, respectively. The air and condensed water moving in the first management device 2a and the air and condensed water moving in the second management device 2b move along different paths, thereby achieving accurate control intended in each of the first management device 2a and the second management device 2b. In this case, since the first management device 2a and the second management device 2b share and use the steam generator 700, the steam generator 700 can be efficiently utilized in the shoes care device 1, and the shoes care device 1 can efficiently utilize the space.

In addition, as described above, when the shoes are dried in one of the first management device 2a and the second management device 2b, the dehumidifying part 330 may be regenerated in the other, and the efficient management of the shoes and efficient use of the shoes care device 1 can be achieved.

In the shoes care device 1 according to one embodiment of the present invention, the first management device 2a and the second management device 2b each include the regeneration path F20 and the damper 350 individually.

The controller 10 may control the heating part 320 (the heater 321) and the damper 350 to interwork with each other.

The controller 10 may control the damper 350 to open the drying flow path F10b and close the regeneration path F20 when the heating part 320 is turned off, and may control the damper 350 to close the drying flow path F10b and open the regeneration path F20 when the heating part 320 is turned on.

The controller 10 may control each component of the shoes care device 1 such that air flowing into the module chamber 210 from the accommodation space 101 and passing through the dehumidifying part 330 moves along the drying flow path F10b when the heating part 320 is turned off (when the heater 321 of the heating part 320 is turned off), and the air moves along the regeneration path F20 when the heating part 320 is turned on (when the heater 321 of the heating part 320 is turned on).

Such control may be performed individually in each of the first management device 2a and the second management device 2b. Accordingly, since the movement path of air inside the module chamber 210 is changed depending on an operation of the heating part 320, the drying of the shoes and the regeneration of the dehumidifying part 330 can be effectively achieved.

The controller 10 may control the steam valve 710 and the heating part 320 to interwork with each other.

The controller 10 ma control the steam valve 710 such that when the heating part 320 of the first management device 2a is turned off, the valve disk 715 closes or opens the first valve outlet 713, when the heating part 320 of the first management device 2a is turned on, the valve disk 715 closes the first valve outlet 713, when the heating part 320 of the second management device 2b is turned off, the valve disk 715 closes or opens the second valve outlet 714, and when the heating part 320 of the second management device 2b is turned on, the valve disk 715 closes the second valve outlet 714.

In this way, the supply of steam to the accommodation space 101 of the inner cabinet 100 and the operation of the heating part 320 inside the module housing 200 may interwork with each other to effectively perform the drying of the shoes and the regeneration of the dehumidifying part 330.

The shoes care device 1 according to one embodiment of the present invention may include a first sensor 361 and a second sensor 362 (see FIG. 9).

The first sensor 361 may be installed in the second module chamber 213 of the module housing 200, and the second sensor 362 may be installed in the third module chamber 214 of the module housing 200. The first sensor 361 may be configured to measure the temperature and/or humidity of the second module chamber 213, and the second sensor 362 may measure the temperature and/or humidity of the third module chamber 214.

The first sensor 361 measures the temperature and/or humidity of air before passing through the dehumidifying part 330, and the second sensor 362 measures the temperature and/or humidity of air after passing through the dehumidifying part 330.

The controller 10 may compare the temperature and/or humidity of the second module chamber 213 measured by the first sensor 361 with the temperature and/or humidity of the third module chamber 214 measured by the second sensor 362 to recognize a state and a change of the temperature and/or humidity inside the module housing, and may also check an operation state of the drying module DM.

The controller 10 may recognize the temperature and humidity of the second module chamber 213 measured by the first sensor 361, and the temperature and humidity of the third module chamber 214 measured by the second sensor 362 to recognize a change in the humidity inside the module housing 200. Accordingly, the degree of dehumidification by the dehumidifying part 330 may be checked, and the degree of regeneration of the dehumidifying part 330 may be checked.

In one embodiment, when the drying module DM operates in the moisture absorption mode, the controller 10 may control the drying module DM to stop operating in the moisture absorption mode and operate in the regeneration mode if a humidity change amount of the second module chamber 213 recognized by the first sensor 361 and a humidity change amount of the third module chamber 214 recognized by the second sensor 362 is less than or equal to a reference value.

In one embodiment, when the drying module DM operates in the regeneration mode, the controller 10 may control the drying module DM to stop operating in the regeneration mode if the humidity change amount of the second module chamber 213 recognized by the first sensor 361 and the humidity change amount of the third module chamber 214 recognized by the second sensor 362 is less than or equal to the reference value.

The shoes care device 1 according to one embodiment of the present invention further includes a third sensor 363 capable of measuring the amount of moisture adsorbed to the dehumidifying material 331, and the controller 10 may control the drying module DM to operate the regeneration mode until the amount of moisture measured by the third sensor 363 is less than or equal to a set value.

Specifically, the controller 10 may control all the heating parts 320 to operate until the amount of moisture measured by the third sensor 363 is less than or equal to the set value.

In this case, as illustrated in FIG. 11, the third sensor 363 may include a moisture sensor installed adjacent to the dehumidifying material 331 to measure the amount of moisture adsorbed to the dehumidifying material 331, and the type and number thereof may vary as necessary.

In this way, when the moisture adsorbed on the dehumidifying material 331 is sensed to exceed the reference value, the shoes care device 1 according to one embodiment of the present invention first regenerates all dehumidifying materials 331 until the moisture is less than or equal to the reference value. Accordingly, the dehumidifying material 331 can maintain an appropriate state for dehumidification all the times even when the shoes care device 1 operates to refresh the shoes.

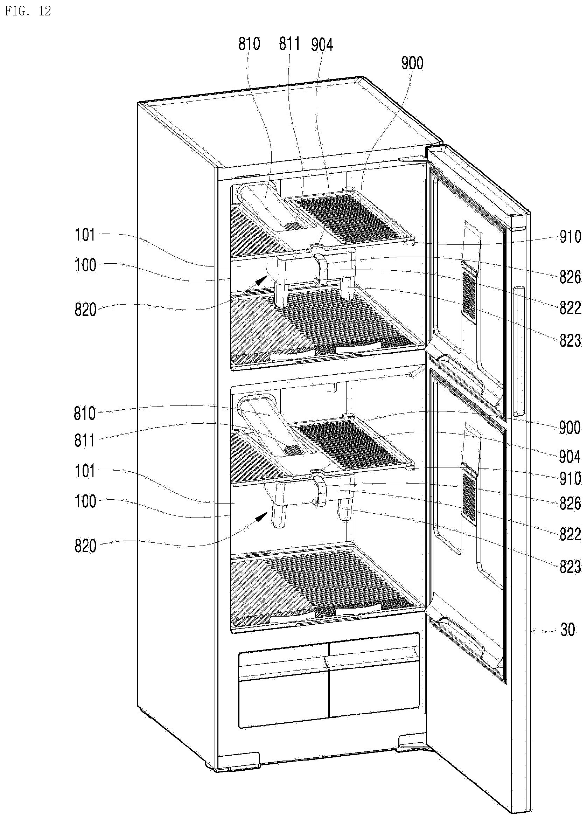

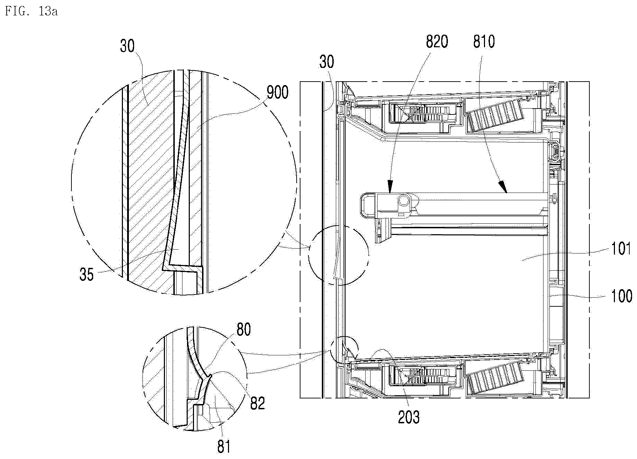

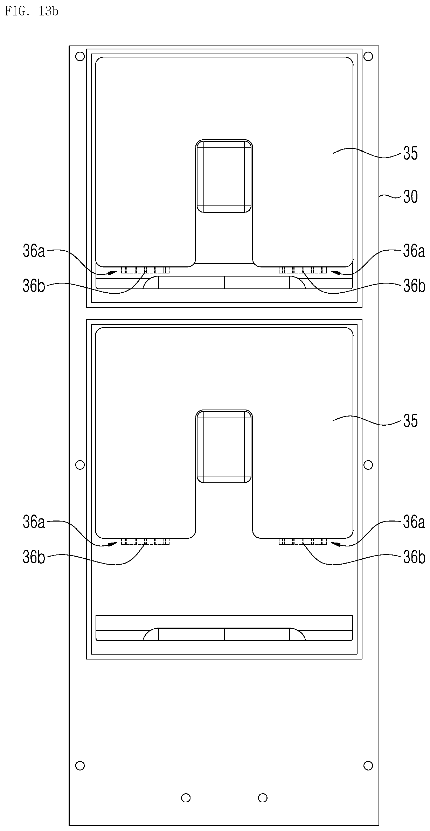

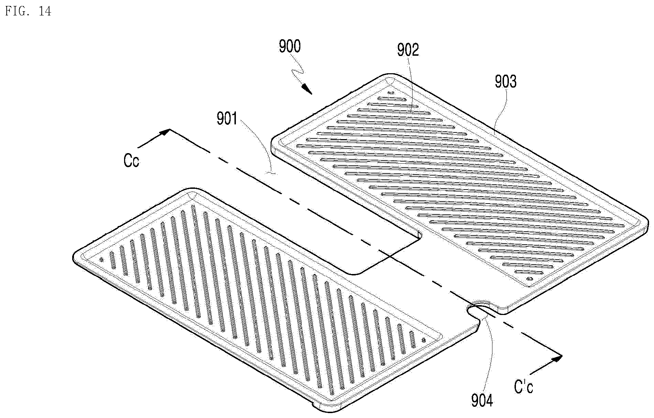

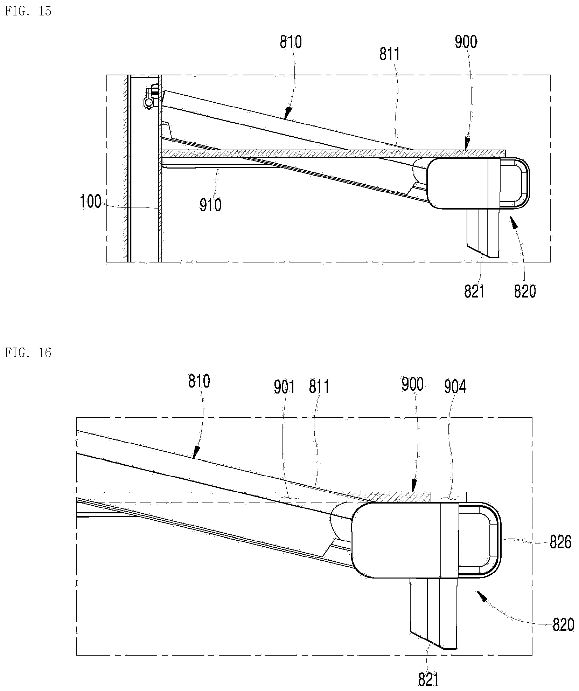

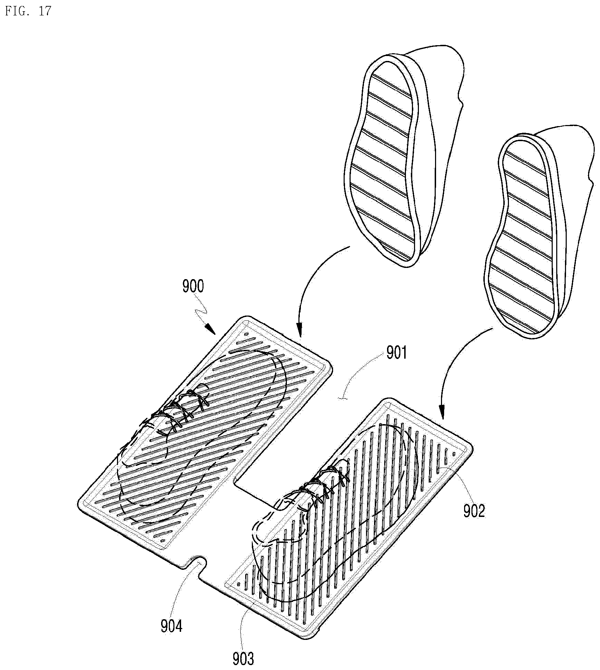

FIG. 12 is a view illustrating a state in which the auxiliary shelf 900 is installed in the shoes care device 1 according to one embodiment of the present invention. FIG. 13a is a cross-sectional view illustrating an inner surface of the door illustrated in the shoes care device according to one embodiment of the present invention. FIGS. 13b to 13e are a view illustrating a insertion groove and a insertion protrusion in more detail in the shoes care device according to one embodiment of the present invention. FIG. 14 is a perspective view illustrating the auxiliary shelf 900 in the shoes care device 1 according to one embodiment of the present invention. FIG. 15 is a view illustrating the auxiliary shelf 900 and the nozzle duct 810 in the shoes care device 1 according to one embodiment of the present invention. FIG. 16 is a view illustrating a state in which the nozzle duct 810 is coupled to a cross section taken along line Cc-C'c of the auxiliary shelf 900 illustrated in FIG. 14. FIG. 17 is a view exemplarily illustrating a state in which the shoes are settled on the auxiliary shelf 900 in the shoes care device 1 according to one embodiment of the present invention.

The shoes care device 1 according to one embodiment of the present invention may include the inner cabinet 100, the outlet 203, the connection path F10, the blowing part 310, the dehumidifying part 330, and the auxiliary shelf 900.

As illustrated in FIG. 12, a shelf holder 910 is formed in the inner cabinet 100, and the auxiliary shelf 900 may be mounted on the shelf holder 910.

In this case, the auxiliary shelf 900 is installed to be mounted on the shelf holder 910, which is a part where the shoes can be settled on the upper surface thereof, and may spatially partition the accommodation space 101 of the inner cabinet 100.

Accordingly, the shoes may be accommodated in each space partitioned by the auxiliary shelf 900 in the inner cabinet 100 and then treated.

As described above, since the shoes care device 1 according to one embodiment of the present invention is used by mounting the auxiliary shelf 900 on the shelf holder 910 formed in the inner cabinet 100, a plurality of shoes can be efficiently treated even in one inner cabinet 100.

In the shoes care device 1 according to one embodiment of the present invention, the auxiliary shelf 900 may be installed to be detachable from the shelf holder 910. That is, the auxiliary shelf 900 may be configured to be separated from the shelf holder 910 in an outer direction of the inner cabinet 100.

Accordingly, when the foreign substances are accumulated on the auxiliary shelf 900 and needs to be removed, or when contaminants on the auxiliary shelf 900 needs to be removed, the user can separate the auxiliary shelf 900 from the inner cabinet 100 and perform cleaning and cleaning.

In addition, even if the auxiliary shelf 900 needs to be inspected and replaced, a predetermined operation may be performed after the auxiliary shelf 900 is separated from the inner cabinet 100.

As described above, in the shoes care device 1 according to one embodiment of the present invention, since the auxiliary shelf 900 is detached from the shelf holder 910, the auxiliary shelf 900 may be easily managed.

In the shoes care device 1 according to one embodiment of the present invention, the auxiliary shelf 900 may have an auxiliary isolation rib 902 formed to protrude upwards from an upper surface thereof.

That is, as illustrated in FIG. 14, the auxiliary isolation rib 902 protrudes from the upper surface of the auxiliary shelf 900, and the bottom surfaces of the shoes may be settled on the auxiliary isolation rib 902.

When the shoes are settled on the upper surface of the planar auxiliary shelf 900, the upper surface of the auxiliary shelf 900 and the bottom surfaces of the shoes are mostly in close contact with each other. In this case, since it is difficult for air to get in contact with the bottom surfaces of the shoes, there may be any difficulty treating the bottom surfaces of the shoes. Further, when water is present between the auxiliary shelf 900 and the shoes, it may be difficult to move or evaporate such water.

Therefore, preferably, a separation space through which air or water may move between the upper surface of the auxiliary shelf 900 and the bottom surfaces of the shoes.

As described above, in the shoes care device 1 according to one embodiment of the present invention, since the auxiliary isolation rib 902 is formed on the upper surface of the auxiliary shelf 900, the separation space for a movement of air or water may be formed between the upper surface of the auxiliary shelf 900 and the bottom surfaces of the shoes.

In the shoes care device 1 according to one embodiment of the present invention, the auxiliary isolation rib 902 may be formed in an oblique direction between the front surface and the rear surface of the inner cabinet 100.

That is, as illustrated in FIGS. 14 and 17, the auxiliary isolation ribs 902 may be formed in the oblique direction so as not to be parallel to the horizontal or vertical direction of the planar auxiliary shelf 900.

In general, the protrusion structure for preventing slipping may be formed on the bottom surfaces of the shoes, and such a protrusion structure may be formed on the bottom surfaces of the shoes along a transverse direction and/or a vertical direction.

Accordingly, when the auxiliary isolation rib 902 is formed horizontally or vertically on the auxiliary shelf 900, a relatively large part may be in parallel with a direction of the protrusion structure formed on the bottom surfaces of the shoes.

In this case, when the protrusion structure formed on the bottom surfaces of the shoes engages with the auxiliary isolation rib 902, the shoes and the upper surface of the auxiliary shelf 900 come into relatively more contact, and as described above, the larger the contact area may result in some disadvantages in terms of the function and maintenance of the shoes care device 1.

Accordingly, the auxiliary isolation rib 902 may be preferably formed in the oblique direction such that the protrusion structure formed on the bottom surfaces of the shoes are not parallel to the auxiliary isolation rib 902 as much as possible.

As described above, in the shoes care device 1 according to one embodiment of the present invention, since the auxiliary isolation rib 902 is formed in the oblique direction, an area in which the upper surface of the auxiliary shelf 900 and the bottom surfaces of the shoes come into contact can be further reduced.

In the shoes care device 1 according to one embodiment of the present invention, the auxiliary shelf 900 may be provided with an auxiliary order rib 903 continuously protruding upwards along the circumferential surface.

That is, as illustrated in FIGS. 14 and 17, the circumferential surface of the auxiliary shelf 900 may be formed to protrude upwards from the remaining parts. Accordingly, water staying on the upper surface of the auxiliary shelf 900 may be blocked by the auxiliary order rib 903, and may be blocked from moving outside the circumferential surface of the auxiliary shelf 900.

In this way, in the shoes care device 1 according to one embodiment of the present invention, since the auxiliary order rib 903 is formed along the circumferential surface of the auxiliary shelf 900, water moving on the auxiliary shelf 900 may be prevented from flowing to an unintended part

In the shoes care device 1 according to one embodiment of the present invention, the auxiliary shelf 900 may be formed in a convex cross-sectional shape from the central part of the inner cabinet 100 to opposite sides. That is, similarly to the main shelf 40, the auxiliary shelf 900 may be formed in a convex shape such that the central part thereof is higher than the opposite sides.

Accordingly, since water staying in the central part of the auxiliary shelf 900 may flow to opposite sides, the occurrence of residual water can be minimized on the auxiliary shelf 900.

In addition, considering the bottom surfaces of shoes formed mostly flat, the upper surface of the auxiliary shelf 900 may have a convex cross-sectional shape, which can minimize a contact area between the upper surface of the auxiliary shelf 900 and the bottom surfaces of the shoes.

In this way, since the central part of the auxiliary shelf 900 is convexly formed, the shoes care device 1 according to one embodiment of the present invention prevents water from accumulating in the central part of the auxiliary shelf 900, and minimizes an area where the upper surface of the auxiliary shelf 900 is in contact with the bottom surfaces of the shoes.

The shoes care device 1 according to one embodiment of the present invention may further include the nozzle duct 810 and the nozzle 820.

The nozzle duct 810 is a part installed inside the inner cabinet 100 and forming a passage of air, and one end thereof is coupled to the inner cabinet 100 and the other thereof end is coupled to the nozzle 820 to form a passage through which air moves to the nozzle 820.

In this case, the nozzle duct 810 may have an upper discharge port 811 opened upwards. For example, since the upper discharge port 811 is formed on a part of the upper surface of the nozzle duct 810, some of the air moving into the nozzle duct 810 may be discharged to the accommodation space 101 through the upper discharge port 811.

Meanwhile, since the nozzle duct 810 is installed to protrude inside the inner cabinet 100, the position where the nozzle 820 is disposed inside the inner cabinet 100 may vary.

The nozzle 820 is a part coupled to the end of the nozzle duct 810 such that the shoes can be inserted in the inner cabinet 100, and air passing through the connection passage F10 may be sprayed into the shoes inside the inner cabinet 100 through the nozzle 820.

Specifically, the nozzle 820 may inject air smoothly into the shoes by injecting air in a state where the nozzle is inserted into the shoes.

In this case, the nozzle 820 may have a lower discharge port 821 opened downwards so as to spray air into the shoes. For example, the lower discharge port 821 is formed in the end of the nozzle 820, and air may be discharged downwards through the lower discharge port 821 in a state where the end of the nozzle 820 is inserted into the shoe.

The nozzle duct 810 may be hinge-coupled to the inner cabinet 100. That is, a nozzle duct hinge axis 813 formed on one end of the nozzle duct 810 is coupled to the inner cabinet 100, and the nozzle duct 810 may be rotated around the nozzle duct hinge axis 813.

The nozzle duct 810 may be installed to protrude ahead from a rear wall of the inner cabinet 100.

Accordingly, depending on whether the nozzle 820 is used or not, the nozzle duct 810 is hinge-rotated inside the inner cabinet 100 and the angle is adjusted such that the nozzle 820 can be displaced in various positions on an inner space of the inner cabinet 100.

The nozzle 820 may be hinge-coupled to the nozzle duct 810. That is, the nozzle 820 may be coupled to a nozzle hinge axis 825 formed in the other end of the nozzle duct 810, and the nozzle 820 may be rotated around the nozzle hinge axis 825.

When the angle of the nozzle duct 810 is changed and the nozzle duct 810 is inclinedly disposed, it may be difficult for the nozzle 820 to get inserted into the upper surfaces of the shoes if the nozzle 820 is also inclined integrally with the nozzle duct 810.

Accordingly, even if the nozzle duct 810 is inclinedly disposed, the angle may be preferably adjusted such that the nozzle 820 is not inclined by more than a predetermined angle.

In this case, the auxiliary shelf 900 may have a cutout portion 901 with a width corresponding to an outer diameter of the nozzle duct 810. That is, as illustrated in FIG. 14, the central part of the auxiliary shelf 900 may be provided with a cutout portion 901 of which one surface is opened and inserted inside.

Further, as illustrated in Figs. 12 and 16, the nozzle duct 810 may be inserted into the cutout portion 901 when the auxiliary shelf 900 is mounted on the shelf holder 910.

That is, in the cutout portion 901 of the auxiliary shelf 900, since a part facing the rear wall of the inner cabinet 100 is opened, the auxiliary shelf 900 may be pushed from the front surface of the inner cabinet 100 to the rear wall.

In this case, since the nozzle duct 810 is inserted into the cutout portion 901 of the auxiliary shelf 900 and the width of the incision 901 corresponds to an outer diameter of the nozzle duct 810, interference by the nozzle duct 810 may not occur during the mounting process of the auxiliary shelf 900.

In the shoes care device 1 according to one embodiment of the present invention, when the auxiliary shelf 900 is mounted in the inner cabinet 100, since the nozzle duct 810 is inserted into the incision 901 of the auxiliary shelf 900, the auxiliary shelf 900 may be mounted while minimizing the interference with the nozzle duct 810.

In the shoes care device 1 according to one embodiment of the present invention, the upper discharge port 811 may be disposed on an upper part of the auxiliary shelf 900 and the lower discharge port 821 may be disposed below the auxiliary shelf 900 based on a state in which the nozzle duct 810 is inserted into the cutout portion 901 and an upper surface of the nozzle 820 is in contact with the auxiliary shelf 900.

That is, as illustrated in FIGS. 12 and 15, when the auxiliary shelf 900 is mounted on the shelf holder 910 to spatially partition the accommodation space 101 of the inner cabinet 100, the upper discharge port 811 may be disposed in an upper space thereof, and the lower discharge port 821 may be disposed in the lower space thereof.

Accordingly, when a plurality of shoes are accommodated in the accommodation space 101 of the inner cabinet 100, the shoes accommodated in the upper shelf 900 may be treated by the air discharged from the upper outlet 811, and the shoes accommodated in the lower shelf 900 may be treated by the air discharged from the lower outlet 821.

As described above, with the auxiliary shelf 900 mounted in the inner cabinet 100, the upper discharge port 811 discharges air to the upper part of the auxiliary shelf 900 and the lower discharge port 821 discharges air to the lower part of the auxiliary shelf 900. Accordingly, the shoes care device 1 according to one embodiment of the present invention can efficiently treat the shoes by placing the shoes above and below the auxiliary shelf 900.

In the shoes care device 1 according to one embodiment of the present invention, the nozzle 820 may include a nozzle body 822 hinge-coupled to the nozzle duct 810 and a nozzle protrusion 823 protruding downwards from the nozzle body 822 and having the lower discharge port 821 formed in the end thereof.

In this case, the upper surface of the nozzle body 822 can be attached to the lower surface of the auxiliary shelf 900 by magnetic force.

The nozzle 820 may be dropped downwards when an unintended external force is applied to the nozzle 820, or the performance of the part supporting the nozzle is degraded due to long-term use.

Accordingly, the upper surface of the nozzle body 822 may be attached to the lower surface of the auxiliary shelf 900 by the magnetic force, and the nozzle 820 may be held on the lower surface of the auxiliary shelf 900.

In this case, a magnetic body may be disposed in at least one of the nozzle body 822 and the auxiliary shelf 900, and the nozzle body 822 and the auxiliary shelf 900 may be made of a material that can be attached by the magnetic force.

In the shoes care device 1 according to one embodiment of the present invention, since the upper surface of the nozzle body 822 and the lower surface of the auxiliary shelf 900 are magnetically attached to each other, the lower surface of the auxiliary shelf 900 and the upper surface of the nozzle body 822 may be attached to each other.

In the shoes care device 1 according to one embodiment of the present invention, the auxiliary shelf 900 may be in close contact with the upper surface of the nozzle duct 810 by inclinedly forming a front lower surface of the cutout portion 901.

That is, as illustrated in FIG. 16, in order to closely adhere the upper surface of the nozzle duct 810 and the lower surface of the auxiliary shelf 900, the front lower surface of the cutout portion 901 may be inclined in a shape corresponding to the upper surface of the nozzle duct 810.

Accordingly, the auxiliary shelf 900 and the nozzle duct 810 can maintain a more stable contact state by minimizing a gap between the lower surface of the auxiliary shelf 900 and the upper surface of the nozzle duct 810.

In this way, in the shoes care device 1 according to one embodiment of the present invention, since the front lower surface of the cutout portion 901 in close contact with the upper surface of the nozzle duct 810 is formed to be inclined, the lower surface of the auxiliary shelf 900 and the upper surface of the nozzle duct 810 can be in stable contact with each other.

In the shoes care device 1 according to one embodiment of the present invention, the nozzle 820 may further include a nozzle handle 826 formed on a front surface of the nozzle body 822, and the auxiliary shelf 900 may have a handle groove 904 formed in a part corresponding to an upper part of the nozzle handle 826.

That is, as illustrated in FIGS. 12 and 16, since the nozzle handle 826 is formed in the nozzle body 822, the user can easily adjust the angle of the nozzle duct 810 by holding the nozzle handle 826 and applying force without touching the nozzle duct 810.

In this case, when the part where the nozzle handle 826 is formed is covered by the auxiliary shelf 900, it may be difficult for the user to recognize the position of the nozzle handle 826 or grasp the nozzle handle 826.

Accordingly, since the handle groove 904 is formed in a part of the auxiliary shelf 900 corresponding to the upper part where the nozzle handle 826 is formed, the user can more easily recognize the position the nozzle handle 826, or grasp the nozzle handle 826.

In this way, in the shoes care device 1 according to one embodiment of the present invention, since the handle groove 904 is formed in the part of the auxiliary shelf 900 corresponding to the upper part of the nozzle handle 826, the angle of the nozzle can be more easily changed through the nozzle handle 826.

The shoes care device 1 according to one embodiment of the present invention may further include the door 30, and the door 30 may include an accommodation groove 35 formed on the inner surface to correspond to the planar shape of the auxiliary shelf 900 and capable of accommodating the auxiliary shelf 900.

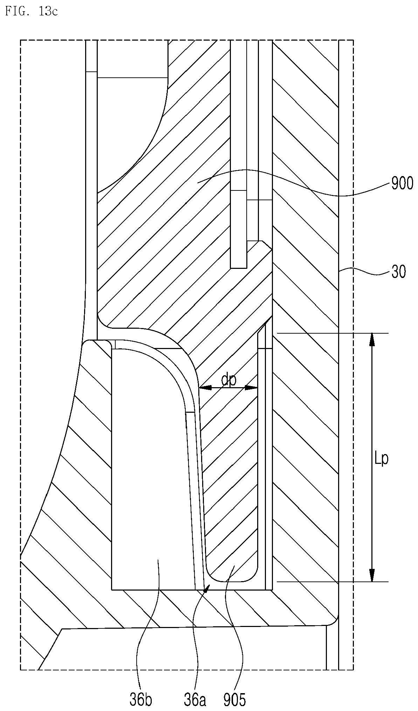

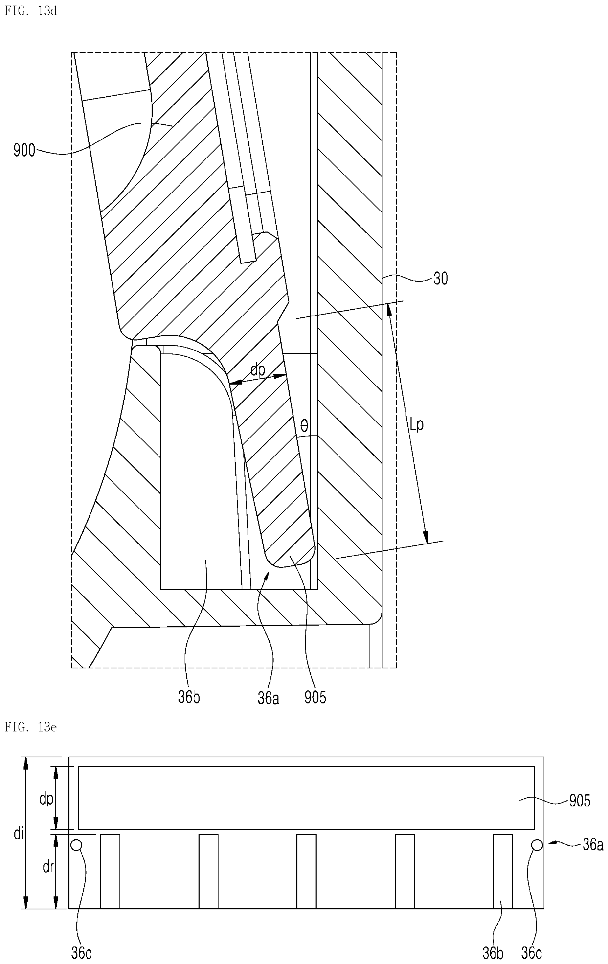

That is, as illustrated in FIG. 13, since the accommodation groove 35 is formed on the inner surface of the door 30, the auxiliary shelf 900 in an unused state may be accommodated in the accommodation groove 35.

As described above, the auxiliary shelf 900 is a component configured to simultaneously perform treatment on several shoes in one inner cabinet 100, and may not be used during the treatment of one shoe.

In this case, since storing the auxiliary shelf 900 in a separate space may be considerably cumbersome from the user's point of view, the auxiliary shelf 900 can be preferably accommodated in the shoes care device 1.

Accordingly, since the shoes care device 1 according to one embodiment of the present invention has the accommodation groove 35 formed on the inner surface of the door 30 to accommodate the auxiliary shelf 900, the auxiliary shelf 900 in the unused state can be conveniently and effectively stored.

In the shoes care device 1 according to one embodiment of the present invention, the accommodation groove 35 may be formed such that the lower part thereof is spaced apart from the auxiliary shelf 900 in a state where the auxiliary shelf 900 is accommodated.

That is, as illustrated in FIG. 13, a predetermined gap may be formed between the auxiliary shelf 900 and the upper part of the accommodation groove 35.

As described above, when the condensed water flows down along the inner surface of the door 30, the condensed water may remain in the accommodation groove 35. Specifically, when both the auxiliary shelf 900 and the accommodation groove 35 are in close contact with each other, bacteria and mold may be generated by the condensed water remaining therebetween.

Accordingly, preferably, as the lower part of the accommodation groove 35 is spaced apart from the auxiliary shelf 900 by the predetermined gap, the condensed water flowing down to the lower part of the accommodation groove 35 is made to evaporate when air is circulated.

In addition, when the user wants to use the auxiliary shelf 900 accommodated in the accommodation groove 35, it may be difficult for the user to separate the auxiliary shelf 900 from the accommodation groove 35 if both the auxiliary shelf 900 and the accommodation groove 35 are in close contact with each other.

Accordingly, as the lower part of the accommodation groove 35 is spaced apart from the auxiliary shelf 900 by the predetermined gap, when the auxiliary shelf 900 is pushed into the corresponding gap, the upper part of the auxiliary shelf 900 is made to protrude from the accommodation groove 35 so that the user can easily separate the auxiliary shelf 900 from the accommodation groove 35.

Since the lower part of the accommodation groove 35 is spaced apart from the auxiliary shelf 900 by the predetermined gap, the shoes care device 1 according to one embodiment of the present invention may prevent moisture from remaining and causing contamination between the auxiliary shelf 900 and the accommodation groove 35, and may easily detach the auxiliary shelf 900 from the accommodation groove 35.

In the shoes care device 1 according to one embodiment of the present invention, the auxiliary shelf 900 may be attached to the accommodation groove 35 by the magnetic force.

The auxiliary shelf 900 in the unused state needs to be stably accommodated in the accommodation groove 35, and needs to be prevented from being unintentionally detached from the accommodation groove 35 even in vibration and impact caused by opening and closing the door 30.

Accordingly, the auxiliary shelf 900 may be attached to the accommodation groove 35 by the magnetic force, and the auxiliary shelf 900 in the unused state may be held in the accommodation groove 35.

In this case, a magnetic material may be disposed in at least one of the accommodation groove 35 and the auxiliary shelf 900, and the accommodation groove 35 and the auxiliary shelf 900 may be made of a material that can be attached by the magnetic force.

In this way, in the shoes care device 1 according to one embodiment of the present invention, since the auxiliary shelf 900 and the accommodation groove 35 are attached to each other by the magnetic force, the auxiliary shelf 900 can be stably attached to the accommodation groove 35.

In the shoes care device 1 according to one embodiment of the present invention, the auxiliary shelf 900 may be accommodated in the accommodation groove 35 in a shape where the cutout portion 901 is disposed in the lower part thereof.

That is, the auxiliary shelf 900 having a 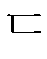 -shaped plane by forming cutout portion 901 may be accommodated in the accommodation groove 35 in a shape where an open surface of the cutout portion 901 is disposed in the lower part thereof. Accordingly, the auxiliary shelf 900 may be accommodated in the accommodation groove 35 in a shape where the handle groove 904 formed on the auxiliary shelf 900 is disposed in the upper part thereof.

As described above, preferably, the lower part of the accommodation groove 35 is spaced apart from the auxiliary shelf 900 by the predetermined gap such that the condensed water flowing down to the lower part of the accommodation groove 35 is evaporated when air is circulated.

Accordingly, the cutout portion 901 with a relatively large open area is disposed in the lower part of the accommodation groove 35 so that the lower part of the accommodation groove 35 can has a larger separation part that that of the auxiliary shelf 900.

In addition, when the user wants to take out the auxiliary shelf 900 from the accommodation groove 35, a handle portion that is easy to grip may be disposed on the upper part, which can be more conveniently used in the user's point of view.

As described above, since the shoes care device 1 according to one embodiment of the present invention accommodates the auxiliary shelf 900 in the accommodation groove 35 such that the cutout portion 901 is disposed downwards, a more separation part is secured between the lower part of the accommodation groove 35 and the auxiliary shelf 900, and the auxiliary shelf 900 can be easily separated from the accommodation groove 35 through the handle groove 904.

The shoe care device 1 according to an embodiment of the present disclosure may further include a door 30, and the door 30 may include an accommodation groove 35 provided on the inner surface to correspond to the planar shape of the auxiliary shelf 900 to be capable of the auxiliary shelf 900, and an insertion groove 36a provided at the lower end of the accommodation groove 35 in an open-top shape.

In this case, the auxiliary shelf 900 may have an insertion protrusion 905 protruding from the lower end in the state in which the auxiliary shelf 900 is accommodated in the accommodation groove 35 to be insertable into the insertion groove 36a.

As described above, the auxiliary shelf 900 may be accommodated in the accommodation groove 35 provided on the inner surface of the door 30 when the auxiliary shelf is not in use. However, the auxiliary shelf 900 may be unintentionally separated from the accommodation groove 35 due to shock, vibration, or the like that may occur in the process of opening and closing the door 30.

Therefore, except for the case where the auxiliary shelf 900 is intentionally separated from the door 30, it may be desirable to ensure that the auxiliary shelf 900 is accommodated stably in the accommodation groove 35 by allowing the insertion protrusion 905 to be inserted into the insertion groove 36a when the auxiliary shelf 900 is accommodated in the accommodation groove 35.

In this way, since the shoe care device 1 according to the present embodiment is configured such that the insertion protrusion 905 of the auxiliary shelf 900 is inserted into the insertion groove 36a in the door 30 while the auxiliary shelf 900 is accommodated in the accommodation grove 35, it is possible to prevent the auxiliary shelf 900 from being unintentionally separated from the accommodation groove 35.

Meanwhile, an insertion groove drain hole 36c configured to discharge condensed water may be provided inside the insertion groove 36a so as to ensure that the condensed water generated due to steam or the like flowing into the auxiliary shelf 900 accommodated in the accommodation groove 35 can be smoothly discharged.

In the shoe care device 1 according to an embodiment of the present disclosure, the insertion groove 36a may have an insertion rib 36b protruding from one surface toward the other opposite surface inside the insertion groove.

In this case, the insertion protrusion 905 may be inserted into the space between the insertion rib 36b and the other surface inside the insertion groove 36a.

That is, as illustrated in FIGS. 13B to 13E, one surface of the insertion protrusion 905 inserted into the insertion groove 36a may be in contact with the other surface of the insertion groove 36a. In addition, the other surface of the insertion protrusion 905 may be in contact with the insertion rib 36b protruding from the one surface of the insertion groove 36a.

Therefore, even when movement occurs in the insertion protrusion 905 inserted into the insertion groove 36a, the movement may be restricted since the insertion protrusion 905 is supported between the insertion rib 36b and the other surface inside the insertion groove 36a.

In this way, since the shoe care device 1 according to the present embodiment is configured such that the insertion groove 36a provided inside the insertion groove 36a supports the insertion protrusion 905 of the auxiliary shelf 900, the insertion protrusion 905 can be supported more stably.

In this case, the insertion rib 36b may be provided to be continuous along the longitudinal direction of the insertion groove 36a. However, in consideration of a coring structure or the like, it may be desirable to provide a plurality of insertion ribs 36b which are discontinuous along the longitudinal direction of the insertion groove 36a.

In the shoe care device 1 according to an embodiment of the present disclosure, the width di of the insertion groove 36a may be relatively larger than the sum of the thickness dp of the insertion protrusion 905 and the thickness dr of the insertion rib 36b.

That is, as illustrated in FIG. 13e, since the gap between the insertion rib 36b and the other surface inside the insertion groove 36a is relatively larger than the thickness dp of the insertion protrusion 905, a certain amount of free space may be formed in the portion of the insertion groove 36a where the insertion protrusion 905 is inserted.

When there is not a certain amount of free space in the portion of the insertion groove 36a where the insertion protrusion 905 is inserted, it may not be easy to insert the insertion protrusion 905 into the insertion groove 36a.

In addition, in order to separate the auxiliary shelf 900 from the accommodation groove 35, it is necessary to tilt the auxiliary shelf 900 at a predetermined angle. However, when there is not a certain amount of free space in the portion of the insertion groove 36a where the insertion protrusion 905 is inserted, it may not be easy to separate the auxiliary shelf 900 since the auxiliary shelf 900 cannot be tilted.

Therefore, it may be desirable to form the width di of the insertion groove 36a to be relatively larger than the sum of the thickness dp of the insertion protrusion 905 and the thickness dr of the insertion rib 36b such that the auxiliary shelf 900 can be tilted at a predetermined angle θ (e.g., 5° to 7°) even when the insertion protrusion 905 is inserted into the insertion groove 36a.

In this way, in the shoe care device 1 according to the present embodiment, since a certain amount of free space is formed in the portion of the insertion groove 36a where the insertion protrusion 905 is inserted, the auxiliary shelf 900 can be tilted at the predetermined angle even when the insertion protrusion 905 is inserted into the insertion groove 36a.

In the shoe care device 1 according to an embodiment of the present disclosure, the length Lp of the insertion protrusion 905 may be relatively larger than the thickness dp of the insertion protrusion 905.

That is, as illustrated in FIGS. 13C and 13D, since the insertion depth of the insertion protrusion 905 is relatively larger than the thickness of the insertion protrusion 905, the insertion protrusion 905 can be inserted into the insertion groove 36a sufficiently deep compared to the width of the insertion groove.

As described above, when the auxiliary shelf 900 is tilted at the predetermined angle θ even when the insertion protrusion 905 is inserted into the insertion groove 36a, there is a risk that the insertion protrusion 905 may be separated from the insertion groove 36a in the state in which the user does not fully separate the auxiliary shelf 900 from the accommodation groove 35.

Therefore, it is necessary to secure a sufficient insertion depth of the insertion protrusion 905 when the auxiliary shelf 900 is tilted at the predetermined angle θ.

Through this, as illustrated in FIG. 13D, when the auxiliary shelf 900 is tilted at the predetermined angle 8, the lower end of the insertion protrusion 905 is supported on the other surface inside the insertion groove 36a and the upper end of the insertion protrusion 905 is supported on the insertion rib 36b, thereby limiting the tilt of the auxiliary shelf 900 and preventing the insertion protrusion 905 from being separated from the insertion groove 36a.

In this way, in the shoe care device 1 according to the present embodiment, since the insertion protrusion 905 is inserted into the insertion groove 36a sufficiently deep compared to the width of the insertion groove, it is possible to prevent the insertion protrusion 90 from being unintentionally separated from the insertion groove 36a even when the auxiliary shelf 900 is tilted at the predetermined angle.

As described above, in the shoe care device 1 according to an embodiment of the present disclosure, since the auxiliary shelf 900 is magnetically attached to the accommodation groove 35, the auxiliary shelf 900 can be stably attached to the accommodation groove 35.

Here, the auxiliary shelf 900 may include a magnetic metal in a portion thereof, and the accommodation groove 35 may include a magnetic material that is capable of attracting the metal of the auxiliary shelf 900.

It may be desirable for the auxiliary shelf 900 to have a certain degree of rigidity in view of the fact that shoes are placed on the upper surface of the auxiliary shelf. Accordingly, the auxiliary shelf 900 may be strengthened in rigidity by partially including metal.

Especially, considering the structure in which the auxiliary shelf 900 and the accommodation groove 35 are magnetically attached to each other, it may be desirable for a portion of the auxiliary shelf 900 to include a magnetic metal. In addition, the accommodation groove 35 may include a magnetic material so that the auxiliary shelf 900 can be magnetically attached to the accommodation groove.

As described above, in the shoe care device 1 according to the present embodiment, since the auxiliary shelf 900 includes metal that can be magnetically attached to the accommodation groove 35, the auxiliary shelf 900 can be strengthened in rigidity while being easily attached to/detached from the accommodation groove 35.

Hereinabove, a specific embodiment of the present invention is described and illustrated, but the present invention is not limited to the disclosed embodiment, and it may be appreciated by those skilled in the art that the exemplary embodiment can be variously modified and transformed to another specific embodiment without departing from the spirit and the scope of the present invention. Therefore, the scope of the present invention will not be defined by the described embodiment, but defined by the technical spirit disclosed in the claims.

### INDUSTRIAL APPLICABILITY

By at least one of exemplary embodiments of the present invention, a dehumidifying part is disposed in a module chamber not only to collect moisture and bacteria in a ventilation air but also to regenerate the dehumidifying part by heating the dehumidifying part in the module chamber, thereby maintaining proper shoes treatment performance all the times.

In addition, by at least one of exemplary embodiments of the present invention, a connection path through which air circulates is formed between a outlet and a nozzle respectively disposed inside an inner cabinet, thereby preventing the air used for dehumidifying and deodorizing shoes from being exposed to the user

In addition, according to at least one of the embodiments of the present disclosure, since an auxiliary shelf is used in the state of being mounted on the shelf holder provided in the inner cabinet, a plurality of shoes can be treated efficiently within only one inner cabinet.

In addition, according to at least one of the embodiments of the present disclosure, since the auxiliary shelf is detachable from the shelf holder, the auxiliary shelf can be easily managed.

In addition, according to at least one of the embodiments of the present disclosure, since the auxiliary isolation rib is provided on the upper surface of the auxiliary shelf, a separation space for the movement of air or water can be formed between the upper surface of the auxiliary shelf and the bottom surfaces of the shoes.

In addition, according to at least one of the embodiments of the present disclosure, since the auxiliary isolation rib is provided in a diagonal direction, the contact area between the upper surface of the auxiliary shelf and the bottom surfaces of the shoes can be further reduced.

In addition, according to at least one of the embodiments of the present disclosure, since the auxiliary order rib is provided along the peripheral surface of the auxiliary shelf, water moving on the auxiliary shelf can be prevented from flowing to an unintended portion.

In addition, according to at least one of the embodiments of the present disclosure, since the central portion of the auxiliary shelf is configured to be convex, water can be prevented from accumulating in the central portion of the auxiliary shelf, and the contact area between the upper surface of the auxiliary shelf and the bottom surfaces of the shoes can be minimized.

In addition, according to at least one of the embodiments of the present disclosure, since the nozzle duct is inserted into the cutout portion in the auxiliary shelf when the auxiliary shelf is mounted in the inner cabinet, the auxiliary shelf can be mounted while minimizing interference with the nozzle duct.

In addition, according to at least one of the embodiments of the present disclosure, since the upper discharge port discharges air to the upper portion of the auxiliary shelf and the lower discharge port discharges air to the lower portion of the auxiliary shelf in the state in which the auxiliary shelf is mounted in the inner cabinet, the shoes can be treated efficiently by being placed in each of the upper and lower portions of the auxiliary shelf.

In addition, according to at least one of the embodiments of the present disclosure, since the upper surface of the nozzle body and the lower surface of the auxiliary shelf are magnetically attached to each other, the lower surface of the auxiliary shelf and the upper surface of the nozzle body can be stably attached to each other.

In addition, according to at least one of the embodiments of the present disclosure, since the front lower surface portion of the cutout portion, which is in close contact with the upper surface of the nozzle duct, is inclined, the lower surface of the auxiliary shelf and the upper surface of the nozzle duct can be stably in close contact with each other.

In addition, according to at least one of the embodiments of the present disclosure, since the handle groove is provided in the auxiliary shelf portion corresponding to the upper portion of the nozzle handle, the angle of the nozzle can be changed more easily by the nozzle handle.

In addition, according to at least one of the embodiments of the present disclosure, since the accommodation groove is provided on the inner surface of the door to accommodate the auxiliary shelf, the auxiliary shelf can be stored conveniently and effectively when not in use.

In addition, according to at least one of the embodiments of the present disclosure, since the lower portion of the accommodation groove is spaced apart from the auxiliary shelf to a certain extent, moisture can be prevented from being contaminated by remaining between the auxiliary shelf and the storage groove, and the auxiliary shelf can be easily detached from the storage groove.

In addition, according to at least one of the embodiments of the present disclosure, since the auxiliary shelf and the accommodation groove are magnetically attached to each other, the auxiliary shelf can be stably attached to the accommodation groove.

In addition, according to at least one of the embodiments of the present disclosure, since the auxiliary shelf is accommodated in the accommodation groove so that the cutout portion is located at the lower side, more space can be secured between the lower portion of the accommodation groove and the auxiliary shelf, and the auxiliary shelf can be easily separated from the accommodation groove through the handle groove located in the upper portion.

In addition, according to at least one of the embodiments of the present disclosure, since the insertion protrusion of the auxiliary shelf is inserted into the insertion groove of the door in the state in which the auxiliary shelf is accommodated in the accommodation groove, the auxiliary shelf can be prevented from being unintentionally separated from the accommodation groove.

In addition, according to at least one of the embodiments of the present disclosure, since the insertion rib provided inside the insertion groove supports the insertion protrusion of the auxiliary shelf, the insertion protrusion inserted into the insertion groove can be supported more stably.

In addition, according to at least one of the embodiments of the present disclosure, a certain amount of free space is provided in the insertion groove where the insertion protrusion is inserted, the auxiliary shelf can be tilted at a predetermined angle even when the insertion protrusion is inserted into the insertion groove.

In addition, according to at least one of the embodiments of the present disclosure, since the insertion protrusion inserted into the insertion groove is inserted sufficiently deeply compared to its width, the insertion protrusion can be prevented from being unintentionally separated from the insertion groove even if the auxiliary shelf is tilted at a predetermined angle.

In addition, according to at least one of the embodiments of the present disclosure, since the auxiliary shelf is made to include a metal that can be magnetically attached to the accommodation groove, the auxiliary shelf can be easily attached to or detached from the accommodation groove, and the rigidity of the auxiliary shelf can be strengthened.

## Claims

1. A shoe care device comprising:
an inner cabinet having an accommodation space configured to accommodate shoes, wherein a shelf holder is provided on an inner side wall;
an outlet provided in a bottom surface of the inner cabinet to allow air inside the inner cabinet is suctioned;
a connection path constituting a flow path through which the air in the accommodation space is discharged from the inner cabinet through the outlet and then introduced back into the accommodation space;
a blowing part disposed in the connection path to blow air;
a dehumidifying part disposed in the connection path to dehumidify air; and
an auxiliary shelf installed to be mounted on the shelf holder so that the shoes can be seated on an upper surface of the auxiliary shelf.

2. The shoe care device of claim 1, wherein the auxiliary shelf is installed to be detachable from the shelf holder.

3. The shoe care device of claim 1, wherein the auxiliary shelf has an auxiliary isolation rib that protrudes upward from the upper surface thereof.

4. The shoe care device of claim 3, wherein the auxiliary isolation rib is provided in a diagonal direction between front and rear surfaces of the inner cabinet.

5. The shoe care device of claim 3, wherein the auxiliary shelf has an auxiliary order rib that continuously protrudes upward along the circumferential surface.

6. The shoe care device of claim 3, wherein the auxiliary shelf has a cross-sectional shape that is convex from a center of the inner cabinet toward opposite side surfaces.

7. The shoe care device of paragraph 1, further comprising:
a nozzle duct installed inside the inner cabinet to provide an air passage and having an upper discharge port that opens upward; and
a nozzle coupled to an end of the nozzle duct so as to be insertable into the shoes inside the inner cabinet and having a lower discharge port that opens downward to spray air into the shoes,
wherein the auxiliary shelf has a cutout portion having a width corresponding to an outer diameter of the nozzle duct, and
wherein the nozzle duct is inserted into the cutout portion when the auxiliary shelf is mounted on the shelf holder.

8. The shoe care device of claim 7, wherein, with reference to a state in which the nozzle duct is inserted into the cutout portion and an upper surface of the nozzle is in contact with the auxiliary shelf, the upper discharge port is disposed at an upper portion of the auxiliary shelf, and the lower discharge port is disposed at a lower portion of the auxiliary shelf.

9. The shoe care device of claim 8, wherein the nozzle comprises:
a nozzle body hinged to the nozzle duct; and
a nozzle protrusion that protrudes downward from the nozzle body, wherein the lower discharge port is provided at an end of the nozzle body, and
wherein the upper surface of the nozzle body is configured to be attached to a lower surface of the auxiliary shelf by using magnetic force.

10. The shoe care device of claim 8, wherein, in the auxiliary shelf, a lower surface in front of the cutout portion is inclined to be in close contact with the upper surface of the nozzle duct.

11. The shoe care device of claim 9, wherein the nozzle further comprises a nozzle handle provided on a front surface of the nozzle body, and
wherein the auxiliary shelf has a handle groove in a portion corresponding to an upper portion of the nozzle handle.

12. The shoe care device of claim 7, further comprising a door configured to open and close a front surface of the inner cabinet,
wherein the door comprises an accommodation groove provided on an inner surface to correspond to a planar shape of the auxiliary shelf so as to accommodate the auxiliary shelf.

13. The shoe care device of claim 12, wherein the accommodation groove is provided such that its lower portion is spaced apart from the auxiliary shelf when the auxiliary shelf is accommodated.

14. The shoe care device of claim 12, wherein the auxiliary shelf is configured to be attached to the accommodation groove by magnetic force.

15. The shoe care device of claim 12, wherein the auxiliary shelf is accommodated in the accommodation groove in a shape where the cutout portion is located at a lower portion thereof.

16. The shoe care device of claim 2, further comprising a door configured to open and close a front surface of the inner cabinet,
wherein the door comprises:
an accommodation groove provided on an inner surface of the door to correspond to a planar shape of the auxiliary shelf to accommodate the auxiliary shelf; and
an insertion groove provided at a lower end of the accommodation groove in an upwardly open shape, and
wherein, with reference to a state of being accommodated in the accommodation groove, the auxiliary shelf has an insertion protrusion protrudes from a lower end thereof to be inserted into the insertion groove.

17. The shoe care device of claim 16, wherein the insertion groove has an insertion rib protruding from one inner surface toward another surface opposite thereto, and
wherein the insertion protrusion is inserted between the another surface inside the insertion groove and the insertion rib.

18. The shoe care device of claim 17, wherein the insertion groove has a width (di) relatively larger than sum of a thickness (dp) of the insertion protrusion and a thickness (dr) of the insertion rib.

19. The shoe care device of claim 18, wherein the insertion protrusion has a length (Lp) relatively larger than the thickness (dp) of the insertion protrusion.

20. The shoe care device of claim 16, wherein the auxiliary shelf is configured to be attached to the accommodation groove by magnetic force.

21. The shoe care device of claim 20, wherein the auxiliary shelf is partially made of a magnetic metal, and
wherein the accommodation groove comprises a magnetic body configured to be attached to the metal of the auxiliary shelf.
